(19) Europäisches Patentamt
European Patent Office
Office européen des brevets

(11) EP 2 970 209 B1

(12) EUROPEAN PATENT SPECIFICATION

(45) Date of publication and mention of the grant of the patent:
**26.12.2018 Bulletin 2018/52**

(21) Application number: **14769343.6**

(22) Date of filing: **12.03.2014**

(51) Int Cl.:
*C07D 471/04* (2006.01) *A61K 31/519* (2006.01)
*A61P 35/00* (2006.01)

(86) International application number:
**PCT/US2014/024564**

(87) International publication number:
**WO 2014/150925 (25.09.2014 Gazette 2014/39)**

(54) **DEUTERATED PALBOCICLIB WITH IMPROVED METABOLIC STABILITY**

DEUTERIERTES PALBOCICLIB MIT VERBESSERTER METABOLISCHER STABILITÄT

PALBOCICLIB DEUTÉRÉ AVEC UNE STABILITÉ MÉTABOLIQUE AMÉLIORÉE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **15.03.2013 US 201361787540 P**

(43) Date of publication of application:
**20.01.2016 Bulletin 2016/03**

(60) Divisional application:
**18205631.7**

(73) Proprietor: **CoNCERT Pharmaceuticals, Inc.**
**Lexington, MA 02421 (US)**

(72) Inventor: **MORGAN, Adam, J.**
**Lexington, MA 02421 (US)**

(74) Representative: **Potter Clarkson LLP**
**The Belgrave Centre**
**Talbot Street**
**Nottingham NG1 5GG (GB)**

(56) References cited:
**WO-A1-2011/101417**

- **DAVID W. ERY ET AL.: 'Specific inhibition of cyclin-dependent kinase 4/6 by PD 0332991 and associated antitumor activity in human tumor xenografts.' MOLE CULAR CANCER THERAPEUTICS. vol. 3, no. 11, November 2004, pages 1427 - 1438, XP002333887**
- **RICHARD S FINN ET AL.: 'PD 0332991, a selective cyclin D kinase 4/6 inhibito r, preferentially inhibits proliferation of luminal estrogen receptor-positi ve human breast cancer cell lines in vitro.' BREAST CANCER RESEARCH vol. 11, no. 5, 2009, page R77, XP021065288**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 970 209 B1

## Description

RELATED APPLICATIONS

This application claims the benefit of and priority to U.S. Provisional Patent Application No. 61/787,540, filed March 15, 2013.

TECHNICAL FIELD OF THE INVENTION

This invention relates to novel pyrido[2,3-d]pyrimidinones and pharmaceutically acceptable salts thereof. This invention also provides compositions comprising a compound of this invention and such compositions for use in treating diseases and conditions that are beneficially treated by administering a CDK-4 and/or CDK-6 inhibitor.

BACKGROUND OF THE INVENTION

Many current medicines suffer from poor absorption, distribution, metabolism and/or excretion (ADME) properties that prevent their wider use or limit their use in certain indications. Poor ADME properties are also a major reason for the failure of drug candidates in clinical trials. While formulation technologies and prodrug strategies can be employed in some cases to improve certain ADME properties, these approaches often fail to address the underlying ADME problems that exist for many drugs and drug candidates. One such problem is rapid metabolism that causes a number of drugs, which otherwise would be highly effective in treating a disease, to be cleared too rapidly from the body. A possible solution to rapid drug clearance is frequent or high dosing to attain a sufficiently high plasma level of drug. This, however, introduces a number of potential treatment problems such as poor patient compliance with the dosing regimen, side effects that become more acute with higher doses, and increased cost of treatment. A rapidly metabolized drug may also expose patients to undesirable toxic or reactive metabolites.

Another ADME limitation that affects many medicines is the formation of toxic or biologically reactive metabolites. As a result, some patients receiving the drug may experience toxicities, or the safe dosing of such drugs may be limited such that patients receive a suboptimal amount of the active agent. In certain cases, modifying dosing intervals or formulation approaches can help to reduce clinical adverse effects, but often the formation of such undesirable metabolites is intrinsic to the metabolism of the compound.

In some select cases, a metabolic inhibitor will be co-administered with a drug that is cleared too rapidly. Such is the case with the protease inhibitor class of drugs that are used to treat HIV infection. The FDA recommends that these drugs be co-dosed with ritonavir, an inhibitor of cytochrome P450 enzyme 3A4 (CYP3A4), the enzyme typically responsible for their metabolism (see Kempf, D.J. et al., Antimicrobial agents and chemotherapy, 1997, 41(3): 654-60). Ritonavir, however, causes adverse effects and adds to the pill burden for HIV patients who must already take a combination of different drugs. Similarly, the CYP2D6 inhibitor quinidine has been added to dextromethorphan for the purpose of reducing rapid CYP2D6 metabolism of dextromethorphan in a treatment of pseudobulbar affect. Quinidine, however, has unwanted side effects that greatly limit its use in potential combination therapy (see Wang, L et al., Clinical Pharmacology and Therapeutics, 1994, 56(6 Pt 1): 659-67; and FDA label for quinidine at www.accessdata.fda.gov).

In general, combining drugs with cytochrome P450 inhibitors is not a satisfactory strategy for decreasing drug clearance. The inhibition of a CYP enzyme's activity can affect the metabolism and clearance of other drugs metabolized by that same enzyme. CYP inhibition can cause other drugs to accumulate in the body to toxic levels.

A potentially attractive strategy for improving a drug's metabolic properties is deuterium modification. In this approach, one attempts to slow the CYP-mediated metabolism of a drug or to reduce the formation of undesirable metabolites by replacing one or more hydrogen atoms with deuterium atoms. Deuterium is a safe, stable, non-radioactive isotope of hydrogen. Compared to hydrogen, deuterium forms stronger bonds with carbon. In select cases, the increased bond strength imparted by deuterium can positively impact the ADME properties of a drug, creating the potential for improved drug efficacy, safety, and/or tolerability. At the same time, because the size and shape of deuterium are essentially identical to those of hydrogen, replacement of hydrogen by deuterium would not be expected to affect the biochemical potency and selectivity of the drug as compared to the original chemical entity that contains only hydrogen.

Over the past 35 years, the effects of deuterium substitution on the rate of metabolism have been reported for a very small percentage of approved drugs (see, e.g., Blake, MI et al, J Pharm Sci, 1975, 64:367-91; Foster, AB, Adv Drug Res 1985, 14:1-40 ("Foster"); Kushner, DJ et al, Can J Physiol Pharmacol 1999, 79-88; Fisher, MB et al, Curr Opin Drug Discov Devel, 2006, 9:101-09 ("Fisher")). The results have been variable and unpredictable. For some compounds deuteration caused decreased metabolic clearance *in vivo.* For others, there was no change in metabolism. Still others demonstrated increased metabolic clearance. The variability in deuterium effects has also led experts to question or dismiss deuterium modification as a viable drug design strategy for inhibiting adverse metabolism (see Foster at p. 35 and Fisher at p. 101).

The effects of deuterium modification on a drug's metabolic properties are not predictable even when deuterium atoms are incorporated at known sites of metabolism. Only by actually preparing and testing a deuterated drug can one determine if and how the rate of metabolism will differ from that of its non-deuterated counterpart. *See,* for example, Fukuto et al. (J. Med. Chem. 1991, 34, 2871-76). Many drugs have multiple sites where metabolism is possible. The site(s) where deuterium substitution is required and the extent of deuteration necessary to see an effect on metabolism, if any, will be different for each drug.

Palbociclib, also known as PD-0332991, and by the chemical name 6-acetyl-8-cyclopentyl-5-methyl-2-[5-(1-piperazinyl)pyridin-2-ylamino]pyrido[2,3-d]pyrimidin-7(8H)-one is an inhibitor of cyclin-dependent kinase (CDK) 4 and 6.

Palbociclib is currently in phase II human clinical trials for breast cancer, colorectal cancer, germ cell cancer, hepatocellular carcinoma, non-small cell lung cancer, glioblastoma multiform, and liposarcoma. It is in Phase I/II human clinical trials for multiple myeloma. Palbociclib is also in Phase I human clinical trials for acute leukemia, mantle cell lymphoma, and myelodysplasia.

Although palbociclib has shown success in clinical trials, exclusion criteria for those trials indicate that this compound is a substrate for cytochrome CYP3A, thus raising the possibility of drug-drug interaction with CYP3A modulators. Therefore, there is a continuing need for new compounds to treat the aforementioned diseases and conditions.
Fry et al (Mol Cancer Ther 2004; 3(11)) discloses specific inhibitors of cyclin-dependent kinase 4/6 by PD 0332991 and associated antitumor activity in human tumor xenografts.
Finn et al (Breast Cancer Research, vol 11, No. 5) describes that PD 0332991 preferentially inhibits proliferation of luminal estrogen receptor-positive human breast cancer cell lines *in vitro.*
WO 2011/101417 discloses specified deuterated pyrrolopyrimidine compounds as inhibitors of CDK 4/6.

SUMMARY OF THE INVENTION

The present invention meets such need by providing compounds of Formula I:

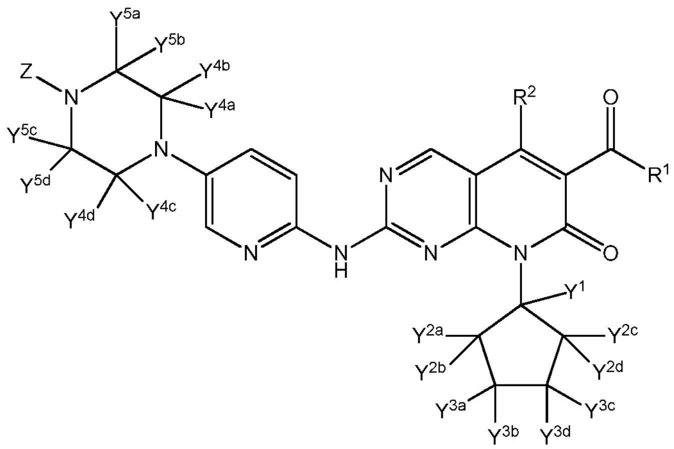

(I),

and pharmaceutically acceptable salts thereof, wherein each of the variables are defined as set forth herein. The invention also provides methods of making and using compounds of Formula I.

DETAILED DESCRIPTION OF THE INVENTION

**Definitions**

The term "treat" means decrease, suppress, attenuate, diminish, arrest, or stabilize the development or progression of a disease (e.g., a disease or disorder delineated herein), lessen the severity of the disease or improve the symptoms associated with the disease.

"Disease" means any condition or disorder that damages or interferes with the normal function of a cell, tissue, or organ.

"The term "alkyl" refers to a monovalent saturated hydrocarbon group. $C_1$-$C_6$ alkyl is an alkyl having from 1 to 6 carbon atoms. An alkyl may be linear or branched. Examples of alkyl groups include methyl; ethyl; propyl, including *n*-propyl and isopropyl; butyl, including *n*-butyl, isobutyl, *sec*-butyl, and *t*-butyl; pentyl, including, for example, *n*-pentyl, isopentyl, and neopentyl; and hexyl, including, for example, *n*-hexyl and 2-methylpentyl.

Unless otherwise specified, "alkylene" by itself or as part of another substituent refers to a saturated straight-chain or branched divalent group having the stated number of carbon atoms and derived from the removal of two hydrogen atoms from the corresponding alkane. Examples of straight chained and branched alkylene groups include $-CH_2-$ (methylene), $-CH_2-CH_2-$ (ethylene), $-CH_2-CH_2-CH_2-$(propylene), $-C(CH_3)_2-$, $-CH_2-CH(CH_3)-$, $-CH_2-CH_2-CH_2-CH_2-$, $-CH_2-CH_2-CH_2-CH_2-CH_2-$ (pentylene), $-CH_2-CH(CH_3)-CH_2-$, and $-CH_2-C(CH_3)_2-CH_2-$.

The term "$C_0$ alkylene" refers to a bond. Thus, when $R^3$, as defined below, is -($C_0$ alkylene)-$C_6$-$C_{10}$ aryl, it is -$C_6$-$C_{10}$ aryl.

"Aryl" by itself or as part of another substituent refers to a monovalent aromatic hydrocarbon group having the stated number of carbon atoms (*i.e.,* $C_5$-$C_{14}$ means from 5 to 14 carbon atoms). Typical aryl groups include, but are not limited to, groups derived from aceanthrylene, acenaphthylene, acephenanthrylene, anthracene, azulene, benzene, chrysene, coronene, fluoranthene, fluorene, hexacene, hexaphene, hexylene, as-indacene, s-indacene, indane, indene, naphthalene, octacene, octophene, octalene, ovalene, penta-2,4-diene, pentacene, pentalene, pentaphene, perylene, phenalene, phenanthrene, picene, pleiadene, pyrene, pyranthrene, rubicene, triphenylene, trinaphthylene, and the like. In a specific embodiment, the aryl group is cyclopentadienyl, phenyl or naphthyl. In a more specific embodiment, the aryl group is phenyl or naphthyl.

It will be recognized that some variation of natural isotopic abundance occurs in a synthesized compound depending upon the origin of chemical materials used in the synthesis. Thus, a preparation of palbociclib will inherently contain small amounts of deuterated isotopologues. The concentration of naturally abundant stable hydrogen and carbon isotopes, notwithstanding this variation, is small and immaterial as compared to the degree of stable isotopic substitution of compounds of this invention. See, for instance, Wada, E et al., Seikagaku, 1994, 66:15; Gannes, LZ et al., Comp Biochem Physiol Mol Integr Physiol, 1998, 119:725.

In the compounds of this invention any atom not specifically designated as a particular isotope is meant to represent any stable isotope of that atom. Unless otherwise stated, when a position is designated specifically as "H" or "hydrogen", the position is understood to have hydrogen at its natural abundance isotopic composition. Also unless otherwise stated, when a position is designated specifically as "D" or "deuterium", the position is understood to have deuterium at an abundance that is at least 3000 times greater than the natural abundance of deuterium, which is 0.015% (i.e., at least 45% incorporation of deuterium).

The term "isotopic enrichment factor" as used herein means the ratio between the isotopic abundance and the natural abundance of a specified isotope.

In other embodiments, a compound of this invention has an isotopic enrichment factor for each designated deuterium atom of at least 3500 (52.5% deuterium incorporation at each designated deuterium atom), at least 4000 (60% deuterium incorporation), at least 4500 (67.5% deuterium incorporation), at least 5000 (75% deuterium), at least 5500 (82.5% deuterium incorporation), at least 6000 (90% deuterium incorporation), at least 6333.3 (95% deuterium incorporation), at least 6466.7 (97% deuterium incorporation), at least 6600 (99% deuterium incorporation), or at least 6633.3 (99.5% deuterium incorporation).

The term "isotopologue" refers to a species in which the chemical structure differs from a specific compound of this invention only in the isotopic composition thereof.

The term "compound," when referring to a compound of this invention, refers to a collection of molecules having an identical chemical structure, except that there may be isotopic variation among the constituent atoms of the molecules. Thus, it will be clear to those of skill in the art that a compound represented by a particular chemical structure containing indicated deuterium atoms, will also contain lesser amounts of isotopologues having hydrogen atoms at one or more of the designated deuterium positions in that structure. The relative amount of such isotopologues in a compound of this invention will depend upon a number of factors including the isotopic purity of deuterated reagents used to make the compound and the efficiency of incorporation of deuterium in the various synthesis steps used to prepare the compound. However, as set forth above the relative amount of such isotopologues *in toto* will be less than 49.9% of the compound. In other embodiments, the relative amount of such isotopologues *in toto* will be less than 47.5%, less than 40%, less than 32.5%, less than 25%, less than 17.5%, less than 10%, less than 5%, less than 3%, less than 1%, or less than 0.5% of the compound.

The invention also provides salts of the compounds of the invention.

A salt of a compound of this invention is formed between an acid and a basic group of the compound, such as an amino functional group, or a base and an acidic group of the compound, such as a carboxyl functional group. According to another embodiment, the compound is a pharmaceutically acceptable acid addition salt.

The term "pharmaceutically acceptable," as used herein, refers to a component that is, within the scope of sound medical judgment, suitable for use in contact with the tissues of humans and other mammals without undue toxicity, irritation, allergic response and the like, and are commensurate with a reasonable benefit/risk ratio. A "pharmaceutically acceptable salt" means any non-toxic salt that, upon administration to a recipient, is capable of providing, either directly or indirectly, a compound of this invention. A "pharmaceutically acceptable counterion" is an ionic portion of a salt that is not toxic when released from the salt upon administration to a recipient.

Acids commonly employed to form pharmaceutically acceptable salts include inorganic acids such as hydrogen

bisulfide, hydrochloric acid, hydrobromic acid, hydroiodic acid, sulfuric acid and phosphoric acid, as well as organic acids such as para-toluenesulfonic acid, salicylic acid, tartaric acid, bitartaric acid, ascorbic acid, maleic acid, besylic acid, fumaric acid, gluconic acid, glucuronic acid, formic acid, glutamic acid, methanesulfonic acid, ethanesulfonic acid, benzenesulfonic acid, lactic acid, oxalic acid, para-bromophenylsulfonic acid, carbonic acid, succinic acid, citric acid, benzoic acid and acetic acid, as well as related inorganic and organic acids. Such pharmaceutically acceptable salts thus include sulfate, pyrosulfate, bisulfate, sulfite, bisulfite, phosphate, monohydrogenphosphate, dihydrogenphosphate, metaphosphate, pyrophosphate, chloride, bromide, iodide, acetate, propionate, decanoate, caprylate, acrylate, formate, isobutyrate, caprate, heptanoate, propiolate, oxalate, malonate, succinate, suberate, sebacate, fumarate, maleate, butyne-1,4-dioate, hexyne-1,6-dioate, benzoate, chlorobenzoate, methylbenzoate, dinitrobenzoate, hydroxybenzoate, methoxybenzoate, phthalate, terephthalate, sulfonate, xylene sulfonate, phenylacetate, phenylpropionate, phenylbutyrate, citrate, lactate, β-hydroxybutyrate, glycolate, maleate, tartrate, methanesulfonate, propanesulfonate, naphthalene-1-sulfonate, naphthalene-2- sulfonate, mandelate and other salts. In one embodiment, pharmaceutically acceptable acid addition salts include those formed with mineral acids such as hydrochloric acid and hydrobromic acid, and especially those formed with organic acids such as maleic acid.

The pharmaceutically acceptable salt may also be a salt of a compound of the present invention having an acidic functional group, such as a carboxylic acid functional group, and a base. Exemplary bases include, but are not limited to, hydroxide of alkali metals including sodium, potassium, and lithium; hydroxides of alkaline earth metals such as calcium and magnesium; hydroxides of other metals, such as aluminum and zinc; ammonia, organic amines such as unsubstituted or hydroxyl-substituted mono-, di-, or tri-alkylamines, dicyclohexylamine; tributyl amine; pyridine; N-methyl, N-ethylamine; diethylamine; triethylamine; mono-, bis-, or tris-(2-OH-($C_1$-$C_6$)-alkylamine), such as N,N-dimethyl-N-(2-hydroxyethyl)amine or tri-(2-hydroxyethyl)amine; N-methyl-D-glucamine; morpholine; thiomorpholine; piperidine; pyrrolidine; and amino acids such as arginine, lysine, and the like.

The compounds of the present invention (e.g., compounds of Formula I), may contain an asymmetric carbon atom, for example, as the result of deuterium substitution or otherwise. As such, compounds of this invention can exist as either individual enantiomers, or mixtures of the two enantiomers. Accordingly, a compound of the present invention may exist as either a racemic mixture or a scalemic mixture, or as individual respective stereoisomers that are substantially free from another possible stereoisomer. The term "substantially free of other stereoisomers" as used herein means less than 25% of other stereoisomers, preferably less than 10% of other stereoisomers, more preferably less than 5% of other stereoisomers and most preferably less than 2% of other stereoisomers are present. Methods of obtaining or synthesizing an individual enantiomer for a given compound are known in the art and may be applied as practicable to final compounds or to starting material or intermediates.

Unless otherwise indicated, when a disclosed compound is named or depicted by a structure without specifying the stereochemistry and has one or more chiral centers, it is understood to represent all possible stereoisomers of the compound.

The term "mammal" as used herein includes a human or a non-human animal, such as mouse, rat, guinea pig, dog, cat, horse, cow, pig, monkey, chimpanzee, baboon, or rhesus. In one embodiment, the mammal is a non-human animal. In another embodiment, the mammal is a human.

The term "stable compounds," as used herein, refers to compounds which possess stability sufficient to allow for their manufacture and which maintain the integrity of the compound for a sufficient period of time to be useful for the purposes detailed herein (e.g., formulation into therapeutic products, intermediates for use in production of therapeutic compounds, isolatable or storable intermediate compounds, treating a disease or condition responsive to therapeutic agents).

"D" and "d" both refer to deuterium. "Stereoisomer" refers to both enantiomers and diastereomers. "Tert" and "t-" each refer to tertiary. "US" refers to the United States of America.

"Substituted with deuterium" refers to the replacement of one or more hydrogen atoms with a corresponding number of deuterium atoms.

Throughout this specification, a variable may be referred to generally (e.g., "each R") or may be referred to specifically (e.g., $R^1$, $R^2$, $R^3$, etc.). Unless otherwise indicated, when a variable is referred to generally, it is meant to include all specific embodiments of that particular variable.

**Therapeutic Compounds**

The present invention provides a compound of Formula I:

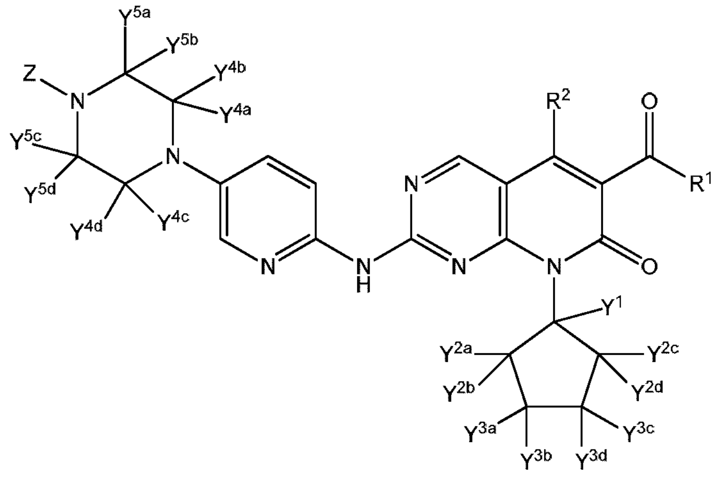

(I),

and pharmaceutically acceptable salts thereof, wherein:

each of $Y^1$, $Y^{2a}$, $Y^{2b}$, $Y^{2c}$, $Y^{2d}$, $Y^{3a}$, $Y^{3b}$, $Y^{3c}$, $Y^{3d}$ is independently hydrogen or deuterium; each of $Y^{4a}$, $Y^{4b}$, $Y^{4c}$, $Y^{4d}$, $Y^{5a}$, $Y^{5b}$, $Y^{5c}$ and $Y^{5d}$ is deuterium;
each of $R^1$ and $R^2$ is independently selected from $CH_3$, $CH_2D$, $CHD_2$ and $CD_3$;
Z is selected from hydrogen, $-C(O)OCH_2OP(O)(OH)_2$, and $-C(O)OCH_2OC(O)CH(R^3)NH_2$;
$R^3$ is selected from hydrogen, $-C_1$-$C_7$ alkyl, and -($C_0$-$C_5$ alkylene)-$C_6$-$C_{10}$ aryl, wherein any alkyl, alkylene or aryl portion of $R^3$ is optionally substituted with -OH.

In certain embodiments of the compound of Formula I, each of $Y^{2a}$ and $Y^{2b}$ is the same; each of $Y^{2c}$ and $Y^{2d}$ is the same; each of $Y^{3a}$ and $Y^{3b}$ is the same; each of $Y^{3c}$ and $Y^{3d}$ is the same. In one aspect of these embodiments, each of $Y^{2a}$, $Y^{2b}$, $Y^{2c}$, and $Y^{2d}$ is the same; each of $Y^{3a}$, $Y^{3b}$, $Y^{3c}$, and $Y^{3d}$ is the same. In one aspect of these embodiments $Y^1$ is hydrogen. In another aspect of these embodiments, $Y^1$ is deuterium. In still another aspect of these embodiments each of $Y^{2a}$, $Y^{2b}$, $Y^{2c}$, and $Y^{2d}$ is deuterium. In an alternate aspect of these embodiments, each of $Y^{2a}$, $Y^{2b}$, $Y^{2c}$, and $Y^{2d}$ is hydrogen. In still another aspect of these embodiments each of $Y^{3a}$, $Y^{3b}$, $Y^{3c}$, and $Y^{3d}$ is deuterium. In an alternate aspect of these embodiments, each of $Y^{3a}$, $Y^{3b}$, $Y^{3c}$, and $Y^{3d}$ is hydrogen.

In certain embodiments of the compound of Formula I, $Y^1$ is hydrogen. In alternate embodiments of Formula I, $Y^1$ is deuterium.

In certain embodiments of the compound of Formula I, each of $R^1$ and $R^2$ is independently selected from $CH_3$ and $CD_3$. In one aspect of these embodiments, $R^2$ is $CD_3$. In an alternate aspect of these embodiments, $R^2$ is $CH_3$. In one aspect of these embodiments, $R^1$ is $CD_3$. In an alternate aspect of these embodiments, $R^1$ is $CH_3$.

In certain embodiments of the compound of Formula I, Z is hydrogen.

In certain embodiments of the compound of Formula I, Z is hydrogen, each of $Y^{2a}$, $Y^{2b}$, $Y^{2c}$, and $Y^{2d}$ is the same; each of $Y^{3a}$, $Y^{3b}$, $Y^{3c}$, and $Y^{3d}$ is the same; $R^1$ is $-CH_3$, and the compound is selected from any one of the compounds (Cmpd) set forth in Table 1a (below):

Table 1a: Exemplary Embodiments of Formula I

| Cmpd # | $Y^1$ | $Y^{2a-d}$ | $Y^{3a-d}$ | $Y^{4a-d}$ | $Y^{5a-d}$ | $R^2$ |
|---|---|---|---|---|---|---|
| 115 | H | H | H | D | D | $CH_3$ |
| 121 | H | D | H | D | D | $CH_3$ |
| 122 | H | H | D | D | D | $CH_3$ |
| 123 | D | D | H | D | D | $CH_3$ |
| 124 | H | D | D | D | D | $CH_3$ |
| 125 | D | D | D | D | D | $CH_3$ |
| 139 | H | H | H | D | D | $CD_3$ |
| 145 | H | D | H | D | D | $CD_3$ |

(continued)

| Cmpd # | $Y^1$ | $Y^{2a-d}$ | $Y^{3a-d}$ | $Y^{4a-d}$ | $Y^{5a-d}$ | $R^2$ |
|---|---|---|---|---|---|---|
| 146 | H | H | D | D | D | $CD_3$ |
| 147 | D | D | H | D | D | $CD_3$ |
| 148 | H | D | D | D | D | $CD_3$ |
| 149 | D | D | D | D | D | $CD_3$ |

wherein any atom not designated as deuterium is present at its natural isotopic abundance, or a pharmaceutically acceptable salt thereof.

In another embodiment, Z is hydrogen, each of $Y^{2a}$, $Y^{2b}$, $Y^{2c}$, and $Y^{2d}$ is the same; each of $Y^{3a}$, $Y^{3b}$, $Y^{3c}$, and $Y^{3d}$ is the same; $R^1$ is $-CD_3$, and the compound is selected from any one of the compounds (Cmpd) set forth in Table 1b (below):

Table 1b: Exemplary Embodiments of Formula I

| Cmpd # | $Y^1$ | $Y^{2a-d}$ | $Y^{3a-d}$ | $Y^{4a-d}$ | $Y^{5a-d}$ | $R^2$ |
|---|---|---|---|---|---|---|
| 165 | H | H | H | D | D | $CH_3$ |
| 171 | H | D | H | D | D | $CH_3$ |
| 172 | H | H | D | D | D | $CH_3$ |
| 173 | D | D | H | D | D | $CH_3$ |
| 174 | H | D | D | D | D | $CH_3$ |
| 175 | D | D | D | D | D | $CH_3$ |
| 195 | H | D | H | D | D | $CD_3$ |
| 196 | H | H | D | D | D | $CD_3$ |
| 197 | D | D | H | D | D | $CD_3$ |
| 198 | H | D | D | D | D | $CD_3$ |
| 199 | D | D | D | D | D | $CD_3$ |

or a pharmaceutically acceptable salt thereof.

In one embodiment, Z is $-C(O)OCH_2OP(O)(OH)_2$, each of $Y^{2a}$, $Y^{2b}$, $Y^{2c}$, and $Y^{2d}$ is the same; each of $Y^{3a}$, $Y^{3b}$, $Y^{3c}$, and $Y^{3d}$ is the same; $R^1$ is $-CH_3$, and the compound is selected from any one of the compounds (Cmpd) set forth in Table 2a (below):

Table 2a: Exemplary Embodiments of Formula I

| Cmpd # | $Y^1$ | $Y^{2a-d}$ | $Y^{3a-d}$ | $Y^{4a-d}$ | $Y^{5a-d}$ | $R^2$ |
|---|---|---|---|---|---|---|
| 215 | H | H | H | D | D | $CH_3$ |
| 221 | H | D | H | D | D | $CH_3$ |
| 222 | H | H | D | D | D | $CH_3$ |
| 223 | D | D | H | D | D | $CH_3$ |
| 224 | H | D | D | D | D | $CH_3$ |
| 225 | D | D | D | D | D | $CH_3$ |
| 245 | H | D | H | D | D | $CD_3$ |
| 246 | H | H | D | D | D | $CD_3$ |
| 247 | D | D | H | D | D | $CD_3$ |
| 248 | H | D | D | D | D | $CD_3$ |

(continued)

| Cmpd # | $Y^1$ | $Y^{2a-d}$ | $Y^{3a-d}$ | $Y^{4a-d}$ | $Y^{5a-d}$ | $R^2$ |
|---|---|---|---|---|---|---|
| 249 | D | D | D | D | D | $CD_3$ |

or a pharmaceutically acceptable salt thereof.

In another embodiment, Z is $-C(O)OCH_2OP(O)(OH)_2$, each of $Y^{2a}$, $Y^{2b}$, $Y^{2c}$, and $Y^{2d}$ is the same; each of $Y^{3a}$, $Y^{3b}$, $Y^{3c}$, and $Y^{3d}$ is the same; $R^1$ is $-CD_3$, and the compound is selected from any one of the compounds (Cmpd) set forth in Table 2b (below):

Table 2b: Exemplary Embodiments of Formula I

| Cmpd# | $Y^1$ | $Y^{2a-d}$ | $Y^{3a-d}$ | $Y^{4a-d}$ | $Y^{5a-d}$ | $R^2$ |
|---|---|---|---|---|---|---|
| 271 | H | D | H | D | D | $CH_3$ |
| 272 | H | H | D | D | D | $CH_3$ |
| 273 | D | D | H | D | D | $CH_3$ |
| 274 | H | D | D | D | D | $CH_3$ |
| 275 | D | D | D | D | D | $CH_3$ |
| 289 | H | H | H | D | D | $CD_3$ |
| 295 | H | D | H | D | D | $CD_3$ |
| 296 | H | H | D | D | D | $CD_3$ |
| 297 | D | D | H | D | D | $CD_3$ |
| 298 | H | D | D | D | D | $CD_3$ |
| 299 | D | D | D | D | D | $CD_3$ |

or a pharmaceutically acceptable salt thereof.

In one embodiment, Z is $-C(O)OCH2OC(O)CH_2NH_2$, each of $Y^{2a}$, $Y^{2b}$, $Y^{2c}$, and $Y^{2d}$ is the same; each of $Y^{3a}$, $Y^{3b}$, $Y^{3c}$, and $Y^{3d}$ is the same; $R^1$ is $-CH_3$, and the compound is selected from any one of the compounds (Cmpd) set forth in Table 3a (below):

Table 3a: Exemplary Embodiments of Formula I

| Cmpd # | $Y^1$ | $Y^{2a-d}$ | $Y^{3a-d}$ | $Y^{4a-d}$ | $Y^{5a-d}$ | $R^2$ |
|---|---|---|---|---|---|---|
| 315 | H | H | H | D | D | $CH_3$ |
| 321 | H | D | H | D | D | $CH_3$ |
| 322 | H | H | D | D | D | $CH_3$ |
| 323 | D | D | H | D | D | $CH_3$ |
| 324 | H | D | D | D | D | $CH_3$ |
| 325 | D | D | D | D | D | $CH_3$ |
| 339 | H | H | H | D | D | $CD_3$ |
| 345 | H | D | H | D | D | $CD_3$ |
| 346 | H | H | D | D | D | $CD_3$ |
| 347 | D | D | H | D | D | $CD_3$ |
| 348 | H | D | D | D | D | $CD_3$ |
| 349 | D | D | D | D | D | $CD_3$ |

or a pharmaceutically acceptable salt thereof.

In another embodiment, Z is $-C(O)OCH_2OC(O)CH_2NH_2$, each of $Y^{2a}$, $Y^{2b}$, $Y^{2c}$ , and $Y^{2d}$ is the same; each of $Y^{3a}$, $Y^{3b}$, $Y^{3c}$, and $Y^{3d}$ is the same; $R^1$ is $-CD_3$, and the compound is selected from any one of the compounds (Cmpd) set forth in Table 3b (below):

Table 3b: Exemplary Embodiments of Formula I

| Cmpd # | $Y^1$ | $Y^{2a-d}$ | $Y^{3a-d}$ | $Y^{4a-d}$ | $Y^{5a-d}$ | $R^2$ |
|---|---|---|---|---|---|---|
| 365 | H | H | H | D | D | $CH_3$ |
| 371 | H | D | H | D | D | $CH_3$ |
| 372 | H | H | D | D | D | $CH_3$ |
| 373 | D | D | H | D | D | $CH_3$ |
| 374 | H | D | D | D | D | $CH_3$ |
| 375 | D | D | D | D | D | $CH_3$ |
| 389 | H | H | H | D | D | $CD_3$ |
| 395 | H | D | H | D | D | $CD_3$ |
| 396 | H | H | D | D | D | $CD_3$ |
| 397 | D | D | H | D | D | $CD_3$ |
| 398 | H | D | D | D | D | $CD_3$ |
| 399 | D | D | D | D | D | $CD_3$ |

or a pharmaceutically acceptable salt thereof.

In another set of embodiments, any atom not designated as deuterium in any of the embodiments set forth above is present at its natural isotopic abundance.

The synthesis of compounds of Formula I may be readily achieved by synthetic chemists of ordinary skill by reference to the Exemplary Synthesis and Examples disclosed herein. Relevant procedures analogous to those of use for the preparation of compounds of Formula I and intermediates thereof are disclosed, for instance in PCT Publication WO 2010/039997.

Such methods can be carried out utilizing corresponding deuterated and optionally, other isotope-containing reagents and/or intermediates to synthesize the compounds delineated herein, or invoking standard synthetic protocols known in the art for introducing isotopic atoms to a chemical structure.

**Exemplary Synthesis**

A convenient method for synthesizing compounds of Formula I is depicted in the following schemes

**Scheme I. Synthesis of Compounds of Formula I wherein Z is hydrogen.**

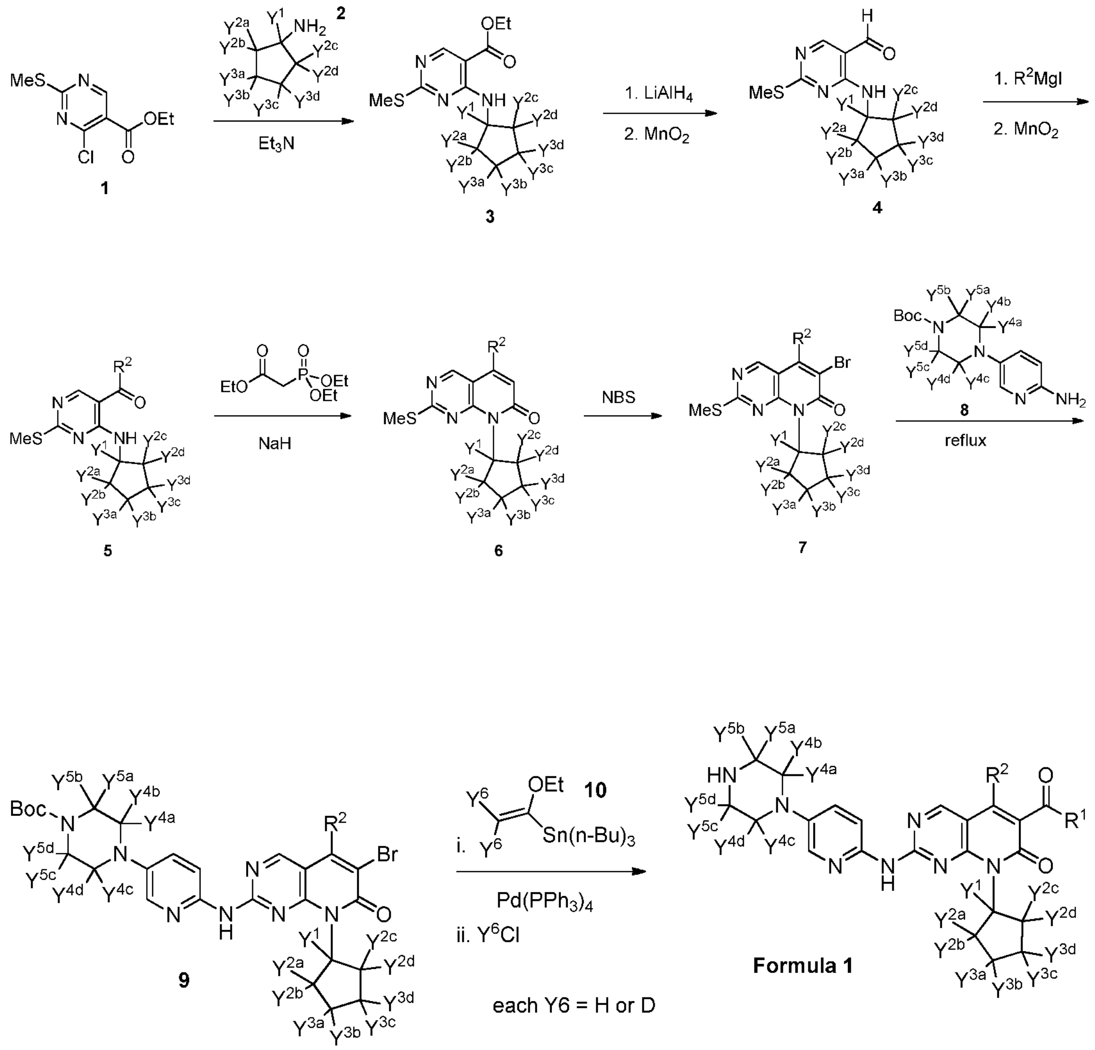


The synthesis of compounds of Formula I is shown in Scheme 1 above and follows the synthetic route described in international patent application WO2010/039997 A2. Reaction of chloropyrimidine **1** with aminocyclopentane **2** provides pyrimidine **3.** Reduction of the ester on pyrimidine **4** and subsequent oxidation affords aldehyde **4** which, in turn, is converted to ketone **5** in two steps. Horner Wadsworth Emmons olefination and subsequent cyclization provides **6** which is then reacted with *N*-bromosuccinimide to afford vinyl bromide **7.** Intermediate **8** is then coupled to **7** under refluxing conditions providing Boc protected piperazine **9.** Stille coupling of **9** with vinyl stannane **10** and subsequent treatment with either HCl or DCl ultimately affords compounds of Formula I.

## Scheme 2: Access to Analogs of Intermediate 2

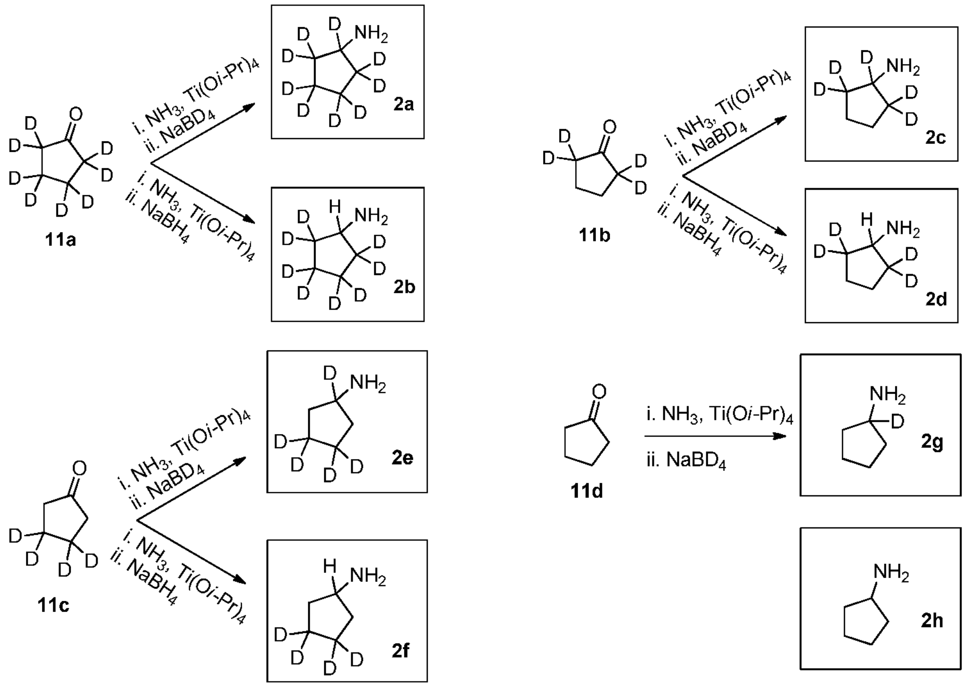


Different analogs of intermediate **2** may be obtained as outlined in Scheme 2 above. Analogs **2a-2g** are obtained via reductive amination of ketones **11a-11d** (**11a-11c** commercially available from CDN Isotopes; **11d** commercially available from Sigma-Aldrich) following the procedure described in Miriyala, B. et al. Tetrahedron 2004, 60, 1463-1471. Analog **2h** is commercially available from Sigma-Aldrich.

## Scheme 3: Synthesis of Intermediate 8

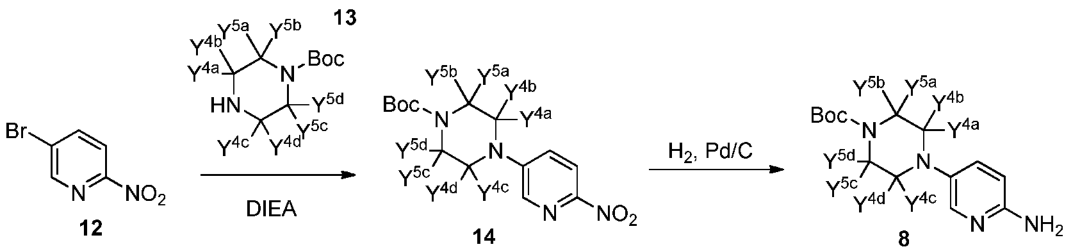


Analogs of intermediate **8** may be obtained as outlined in Scheme 3 shown above following the synthetic route described in international patent application WO 2010039997 A2. Reaction of bromopyridine **12** with Boc-piperazine **13** affords nitropyridine **14.** Palladium catalyzed hydrogenation of **14** ultimately affords Boc-piperazine **8.** Different analogs of Boc-piperazine intermediate **13** are depicted in Scheme 4 below.

## Scheme 4: Analogs of Intermediate 13

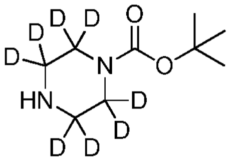
**13a**

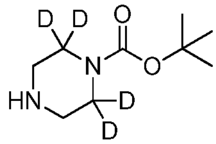
**13b**

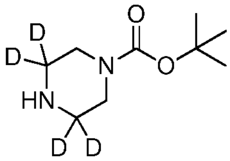
**13c**

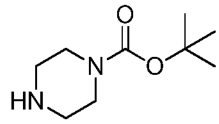
**13d**

Analogs of intermediate **13** useful in the synthesis of intermediate **8** (Scheme 3 above) are readily obtainable.

Boc-piperazines **13a** and **13c** are commercially available from CDN Isotopes. The synthesis of **13b** has been previously described (Dischino, D. D. et al. Journal of Labelled Compounds and Radiopharmaceuticals 1988, 25, 359-367). Boc-piperazine **13d** is commercially available from Sigma-Aldrich.

**Scheme 5a. Synthesis of Compounds of Formula I wherein Z is -C(O)OCH$_2$OP(O)(OH)$_2$.**

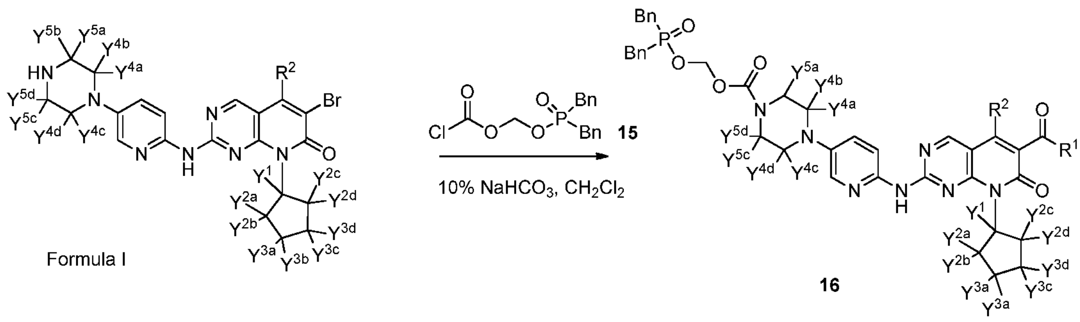


Formula I

16

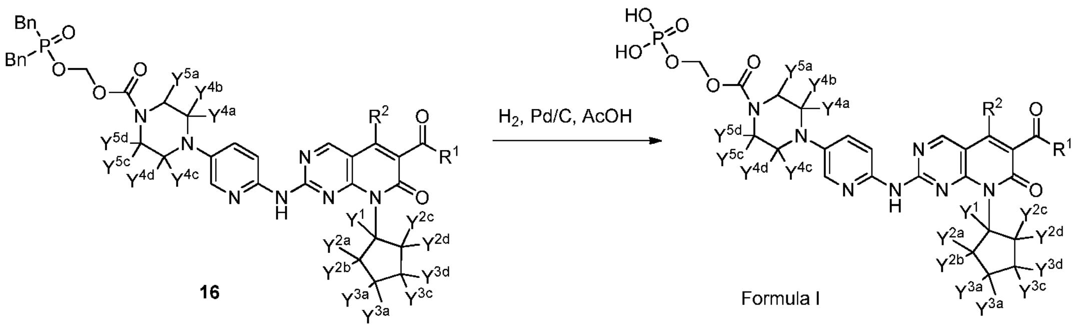


16

Formula I

As shown in Scheme 5a, a compound of Formula I wherein Z is H - obtained, for example, as disclosed in Scheme 1 - is treated with **15** optionally in the presence of a base such as diisopropylethylamine to provide **16** which, following reductive debenzylation, affords the compound of formula I wherein Z is -C(O)OCH$_2$OP(O)(OH)$_2$. Intermediate **15** (bis(benzyloxy)phosphoryloxy)methyl carbonochloridate is known from PCT publication WO2007050732A1 and European Patent publication EP747385. The disodium or calcium salt forms of the compound of formula I thus obtained may be accessed via treatment with sodium hydroxide or with calcium hydroxide or calcium acetate or calcium chloride.

**Scheme 5b. Alternate Synthesis of Compounds of Formula I wherein Z is $-C(O)OCH_2OP(O)(OH)_2$.**

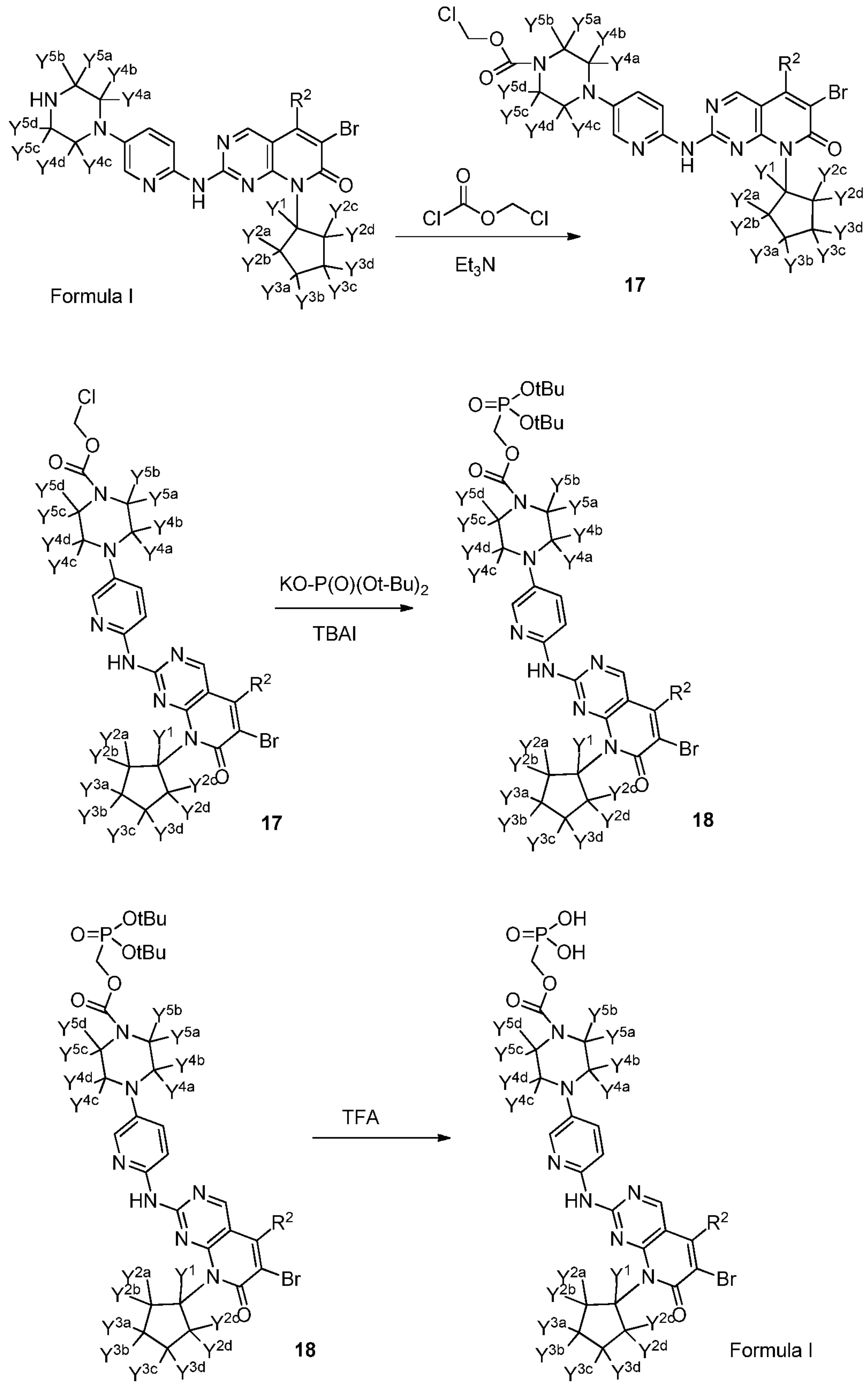

Scheme 5b depicts an alternate method for producing compounds of Formula I where Z is $-C(O)OCH_2OP(O)(OH)_2$. A compound of Formula I wherein Z is hydrogen is treated with chloromethyl chloroformate to provide compound **17.** Treatment with di-tert-butylphosphate potassium salt and tetrabutylammonium iodide (TBAI) provides compound **18.** Removal of the t-butyl groups via treatment with trifluoroacetic acid provides compounds of Formula I wherein Z
is $-C(O)OCH_2OP(O)(OH)_2$. The disodium or calcium salt forms of the compound of formula I thus obtained may be accessed via treatment with sodium hydroxide or with calcium hydroxide or calcium acetate or calcium chloride.

**Scheme 6. Synthesis of Compounds of Formula I wherein Z is $-C(O)OCH_2OC(O)CH(R^3)NH_2$.**

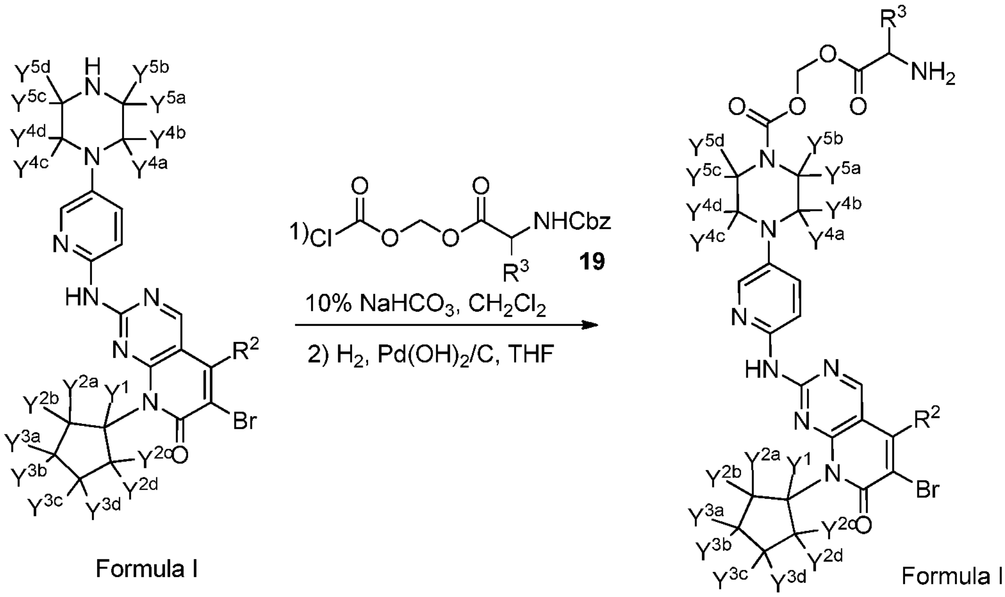


As shown in Scheme 6, a compound of formula I wherein Z is H - obtained, for example, as disclosed in Scheme 1 - is treated with **19,** which may be obtained as disclosed in Scheme 7 below, to provide, after removal of the Cbz protecting group by treatment with hydrogen and $Pd(OH)_2/C$, the compound of formula I wherein Z is $-C(O)OCH_2OC(O)CH(R^3)NH_2$.

**Scheme 7. Preparation of Intermediate 19.**

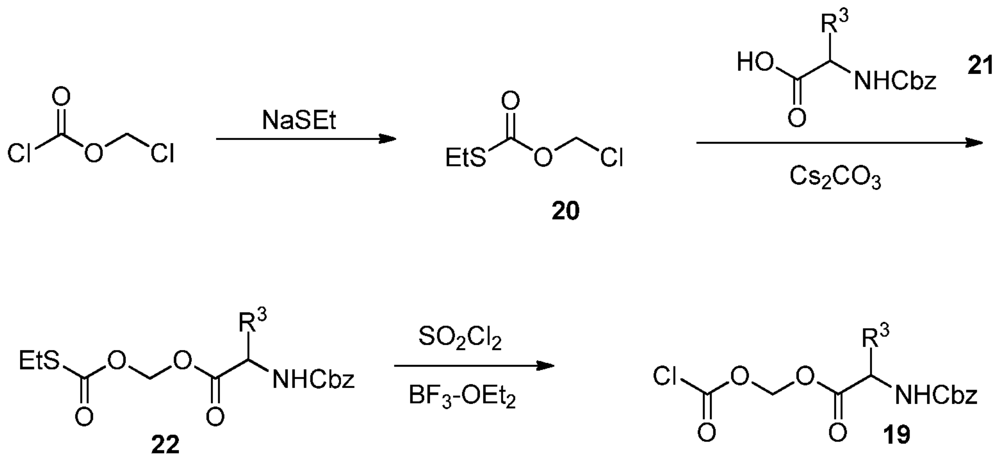


As shown in Scheme 7, Compound **19** may be prepared by treating chloromethyl chloroformate with NaSEt to provide **20;** treating **20** with protected amino acid **21** to give **22;** and reacting **22** with sulfuryl chloride to afford **19.** A variety of naturally occurring amino acids may be envisioned protected amino acid **21,** such as valine, in which case the

corresponding structure of **19** resulting from Scheme 7 is

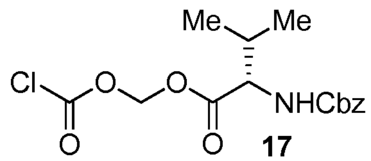

**17**

The specific approaches and compounds shown above are not intended to be limiting. The chemical structures in the schemes herein depict variables that are hereby defined commensurately with chemical group definitions (moieties, atoms, etc.) of the corresponding position in the compound formulae herein, whether identified by the same variable name (i.e., $R^1$, $R^2$, $R^3$, etc.) or not. The suitability of a chemical group in a compound structure for use in the synthesis of another compound is within the knowledge of one of ordinary skill in the art.

Additional methods of synthesizing compounds of Formula I and their synthetic precursors, including those within routes not explicitly shown in schemes herein, are within the means of chemists of ordinary skill in the art. Synthetic chemistry transformations and protecting group methodologies (protection and deprotection) useful in synthesizing the applicable compounds are known in the art and include, for example, those described in Larock R, Comprehensive Organic Transformations, VCH Publishers (1989); Greene, TW et al., Protective Groups in Organic Synthesis, 3rd Ed., John Wiley and Sons (1999); Fieser, L et al., Fieser and Fieser's Reagents for Organic Synthesis, John Wiley and Sons (1994); and Paquette, L, ed., Encyclopedia of Reagents for Organic Synthesis, John Wiley and Sons (1995) and subsequent editions thereof.

Combinations of substituents and variables envisioned by this invention are only those that result in the formation of stable compounds.

**Compositions**

The invention also provides pharmaceutical compositions comprising an effective amount of a compound of Formula I (e.g., including any of the formulae herein), or a pharmaceutically acceptable salt of said compound; and a pharmaceutically acceptable carrier. The carrier(s) are "acceptable" in the sense of being compatible with the other ingredients of the formulation and, in the case of a pharmaceutically acceptable carrier, not deleterious to the recipient thereof in an amount used in the medicament. Such pharmaceutical compositions are typically pyrogen-free.

Pharmaceutically acceptable carriers, adjuvants and vehicles that may be used in the pharmaceutical compositions of this invention include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

If required, the solubility and bioavailability of the compounds of the present invention in pharmaceutical compositions may be enhanced by methods well-known in the art. One method includes the use of lipid excipients in the formulation. See "Oral Lipid-Based Formulations: Enhancing the Bioavailability of Poorly Water-Soluble Drugs (Drugs and the Pharmaceutical Sciences)," David J. Hauss, ed. Informa Healthcare, 2007; and "Role of Lipid Excipients in Modifying Oral and Parenteral Drug Delivery: Basic Principles and Biological Examples," Kishor M. Wasan, ed. Wiley-Interscience, 2006.

Another known method of enhancing bioavailability is the use of an amorphous form of a compound of this invention optionally formulated with a poloxamer, such as LUTROL™ and PLURONIC™ (BASF Corporation), or block copolymers of ethylene oxide and propylene oxide. See United States patent 7,014,866; and United States patent publications 20060094744 and 20060079502.

The pharmaceutical compositions of the invention include those suitable for oral, rectal, nasal, topical (including buccal and sublingual), vaginal or parenteral (including subcutaneous, intramuscular, intravenous and intradermal) administration. In certain embodiments, the compound of the formulae herein is administered transdermally (e.g., using a transdermal patch or iontophoretic techniques). Other formulations may conveniently be presented in unit dosage form, e.g., tablets, sustained release capsules, and in liposomes, and may be prepared by any methods well known in the art of pharmacy. See, for example, Remington: The Science and Practice of Pharmacy, Lippincott Williams & Wilkins, Baltimore, MD (20th ed. 2000).

Such preparative methods include the step of bringing into association with the molecule to be administered ingredients such as the carrier that constitutes one or more accessory ingredients. In general, the compositions are

prepared by uniformly and intimately bringing into association the active ingredients with liquid carriers, liposomes or finely divided solid carriers, or both, and then, if necessary, shaping the product.

In certain embodiments, the compound is administered orally. Compositions of the present invention suitable for oral administration may be presented as discrete units such as capsules, sachets, or tablets each containing a predetermined amount of the active ingredient; a powder or granules; a solution or a suspension in an aqueous liquid or a non-aqueous liquid; an oil-in-water liquid emulsion; a water-in-oil liquid emulsion; packed in liposomes; or as a bolus, etc. Soft gelatin capsules can be useful for containing such suspensions, which may beneficially increase the rate of compound absorption.

In the case of tablets for oral use, carriers that are commonly used include lactose and corn starch. Lubricating agents, such as magnesium stearate, are also typically added. For oral administration in a capsule form, useful diluents include lactose and dried cornstarch. When aqueous suspensions are administered orally, the active ingredient is combined with emulsifying and suspending agents. If desired, certain sweetening and/or flavoring and/or coloring agents may be added.

Compositions suitable for oral administration include lozenges comprising the ingredients in a flavored basis, usually sucrose and acacia or tragacanth; and pastilles comprising the active ingredient in an inert basis such as gelatin and glycerin, or sucrose and acacia.

Compositions suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example, sealed ampules and vials, and may be stored in a freeze dried (lyophilized) condition requiring only the addition of the sterile liquid carrier, for example water for injections, immediately prior to use. Extemporaneous injection solutions and suspensions may be prepared from sterile powders, granules and tablets.

Such injection solutions may be in the form, for example, of a sterile injectable aqueous or oleaginous suspension. This suspension may be formulated according to techniques known in the art using suitable dispersing or wetting agents (such as, for example, Tween 80) and suspending agents. The sterile injectable preparation may also be a sterile injectable solution or suspension in a non-toxic parenterally-acceptable diluent or solvent, for example, as a solution in 1,3-butanediol. Among the acceptable vehicles and solvents that may be employed are mannitol, water, Ringer's solution and isotonic sodium chloride solution. In addition, sterile, fixed oils are conventionally employed as a solvent or suspending medium. For this purpose, any bland fixed oil may be employed including synthetic mono- or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant.

The pharmaceutical compositions of this invention may be administered in the form of suppositories for rectal administration. These compositions can be prepared by mixing a compound of this invention with a suitable non-irritating excipient which is solid at room temperature but liquid at the rectal temperature and therefore will melt in the rectum to release the active components. Such materials include, but are not limited to, cocoa butter, beeswax and polyethylene glycols.

The pharmaceutical compositions of this invention may be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other solubilizing or dispersing agents known in the art. See, e.g.: Rabinowitz JD and Zaffaroni AC, US Patent 6,803,031, assigned to Alexza Molecular Delivery Corporation.

Topical administration of the pharmaceutical compositions of this invention is especially useful when the desired treatment involves areas or organs readily accessible by topical application. For topical application topically to the skin, the pharmaceutical composition should be formulated with a suitable ointment containing the active components suspended or dissolved in a carrier. Carriers for topical administration of the compounds of this invention include, but are not limited to, mineral oil, liquid petroleum, white petroleum, propylene glycol, polyoxyethylene polyoxypropylene compound, emulsifying wax, and water. Alternatively, the pharmaceutical composition can be formulated with a suitable lotion or cream containing the active compound suspended or dissolved in a carrier. Suitable carriers include, but are not limited to, mineral oil, sorbitan monostearate, polysorbate 60, cetyl esters wax, cetearyl alcohol, 2-octyldodecanol, benzyl alcohol, and water. The pharmaceutical compositions of this invention may also be topically applied to the lower intestinal tract by rectal suppository formulation or in a suitable enema formulation. Topically-transdermal patches and iontophoretic administration are also included in this invention.

Application of the subject therapeutics may be local, so as to be administered at the site of interest. Various techniques can be used for providing the subject compositions at the site of interest, such as injection, use of catheters, trocars, projectiles, pluronic gel, stents, sustained drug release polymers or other device which provides for internal access.

Thus, according to yet another embodiment, the compounds of this invention may be incorporated into compositions for coating an implantable medical device, such as prostheses, artificial valves, vascular grafts, stents, or catheters. Suitable coatings and the general preparation of coated implantable devices are known in the art and are exemplified in US Patents 6,099,562; 5,886,026; and 5,304,121. The coatings are typically biocompatible polymeric materials such as a hydrogel polymer, polymethyldisiloxane, polycaprolactone, polyethylene glycol, polylactic acid, ethylene vinyl acetate, and mixtures thereof. The coatings may optionally be further covered by a suitable topcoat of fluorosilicone, polysaccharides, polyethylene glycol, phospholipids or combinations thereof to impart controlled release characteristics in the composition. Coatings for invasive devices are to be included within the definition of pharmaceutically acceptable carrier, adjuvant or vehicle, as those terms are used herein.

According to another embodiment, the invention provides a method of coating an implantable medical device comprising the step of contacting said device with the coating composition described above. It will be obvious to those skilled in the art that the coating of the device will occur prior to implantation into a mammal.

According to another embodiment, the invention provides a method of impregnating an implantable drug release device comprising the step of contacting said drug release device with a compound or composition of this invention. Implantable drug release devices include, but are not limited to, biodegradable polymer capsules or bullets, non-degradable, diffusible polymer capsules and biodegradable polymer wafers.

According to another embodiment, the invention provides an implantable medical device coated with a compound or a composition comprising a compound of this invention, such that said compound is therapeutically active.

According to another embodiment, the invention provides an implantable drug release device impregnated with or containing a compound or a composition comprising a compound of this invention, such that said compound is released from said device and is therapeutically active.

Where an organ or tissue is accessible because of removal from the subject, such organ or tissue may be bathed in a medium containing a composition of this invention, a composition of this invention may be painted onto the organ, or a composition of this invention may be applied in any other convenient way.

In another embodiment, a composition of this invention further comprises a second therapeutic agent. The second therapeutic agent may be selected from any compound or therapeutic agent known to have or that demonstrates advantageous properties when administered with a CDK-4 or CDK-6 inhibitor. Such agents include those indicated as being useful in combination with palbociclib, including but not limited to, those described in WO2003062236, WO2008076946, WO2009014642, WO2009061345, WO2010039997, WO2010051127, WO2010132725, WO2011130232 and WO 2012068381.

In certain embodiments, the second therapeutic agent is selected from an anti-cancer agent (e.g., a chemotherapeutic agent), a therapeutic for the treatment of neurological disease, a purine biosynthesis inhibitor, or an mTOR inhibitor.

In one embodiment, the second therapeutic agent is selected from 5-FU, oxaliplatin, bortezomib, dexamethasone, anastrozole, letrozole, ara-C, mitoxantrone, and paclitaxel.

In another embodiment, the invention provides separate dosage forms of a compound of this invention and one or more of any of the above-described second therapeutic agents, wherein the compound and second therapeutic agent are associated with one another. The term "associated with one another" as used herein means that the separate dosage forms are packaged together or otherwise attached to one another such that it is readily apparent that the separate dosage forms are intended to be sold and administered together (within less than 24 hours of one another, consecutively or simultaneously).

In the pharmaceutical compositions of the invention, the compound of the present invention is present in an effective amount. As used herein, the term "effective amount" refers to an amount which, when administered in a proper dosing regimen, is sufficient to treat the target disorder.

The interrelationship of dosages for animals and humans (based on milligrams per meter squared of body surface) is described in Freireich et al., Cancer Chemother. Rep, 1966, 50: 219. Body surface area may be approximately determined from height and weight of the subject. See, e.g., Scientific Tables, Geigy Pharmaceuticals, Ardsley, N.Y., 1970, 537.

In one embodiment, an effective amount of a compound of this invention can range from 1 mg - 1,000 mg/day. In one aspect of this embodiment an effective amount can range from 5 mg to 250 mg/day. In another aspect of this embodiment an effective amount can be 5 mg/day, 10 mg/day, 15 mg/day, 20 mg/day, 25 mg/day, 50 mg/day, 75 mg/day, 100 mg/day, 125 mg/day, 150 mg/day, 175 mg/day, 200 mg/day, 225 mg/day or 250 mg/day. In certain aspects of any of the foregoing dosage embodiments, the compound of the invention is administered orally. In other aspects of any of the foregoing dosage embodiments, the compound of the invention is administered by intravenous infusion over one, two, three, four, five or six hours. In still other aspects of any of the foregoing dosage embodiments, the compound of the invention is administered to a subject who has not eaten 4, 5, 6, 7, 8, 9, 10, 11, or 12 hours or more prior to administration (e.g., administration on an empty stomach).

In certain embodiments, the compound of the invention is administered once a day for three weeks, followed

by one week of no compound. In other embodiments, the compound of the invention is administered once a day for two weeks, followed by one week of no compound. In other embodiments, the compound of the invention is administered once a day for one week, followed by one week of no compound. In still other embodiments, the compound of the invention is administered once a day for 12 days followed by nine days of no compound. Each of the dosing cycles set forth above may repeated two, three, four, five or more times in a course of treatments.

Effective doses will also vary, as recognized by those skilled in the art, depending on the diseases treated, the severity of the disease, the route of administration, the sex, age and general health condition of the subject, excipient usage, the possibility of co-usage with other therapeutic treatments such as use of other agents and the judgment of the treating physician.

For pharmaceutical compositions that comprise a second therapeutic agent, an effective amount of the second therapeutic agent is between about 20% and 100% of the dosage normally utilized in a monotherapy regime using just that agent. Preferably, an effective amount is between about 70% and 100% of the normal monotherapeutic dose. The normal monotherapeutic dosages of these second therapeutic agents are well known in the art. *See,* e.g., Wells et al., eds., Pharmacotherapy Handbook, 2nd Edition, Appleton and Lange, Stamford, Conn. (2000); PDR Pharmacopoeia, Tarascon Pocket Pharmacopoeia 2000, Deluxe Edition, Tarascon Publishing, Loma Linda, Calif. (2000).

It is expected that some of the second therapeutic agents referenced above will act synergistically with the compounds of this invention. When this occurs, it will allow the effective dosage of the second therapeutic agent and/or the compound of this invention to be reduced from that required in a monotherapy. This has the advantage of minimizing toxic side effects of either the second therapeutic agent of a compound of this invention, synergistic improvements in efficacy, improved ease of use and/or reduced overall expense of compound preparation or formulation.

**Treatment**

According to another embodiment, the invention provides a compound or composition of the invention for use in treating a disease that is beneficially treated by an inhibitor of CDK-4 or CDK-6 in a subject in need thereof. In one embodiment the subject is a patient in need of such treatment. Such diseases are well known in the art and are disclosed in, but not limited to, the following patents and published applications: WO03062236, WO 2009014642, WO 2009061345, WO 2010132725, and WO 2011130232. Such diseases include, but are not limited to, cancer, autoimmune disease and allergies.

In another embodiment, the compounds of the invention are for use as hematopoietic protection agents, neuroprotection agents or renal protection agents. Such uses are disclosed, but not limited to, the following published patent applications: WO 2008076946, WO 2010039997, WO 2010051127 and WO 2012068381. These protective uses may be employed in conjunction with radiation treatments, chemotherapeutic treatments, in the treatment of Alzheimer's Disease and other dementias and other neurodegenerative diseases, and to prevent ischemia-reperfusion injury.

In one particular embodiment, the compound or composition of this invention is for use to treat a disease or condition selected from breast cancer, solid tumors, colorectal cancer, hepatocellular carcinoma, liposarcoma, ovarian cancer, multiple myeloma, acute leukemia, mantle cell lymphoma, myelodysplasia, glioblastoma, and non-small cell lung cancer in a subject in need thereof.

Identifying a subject in need of such treatment can be in the judgment of a subject or a health care professional and can be subjective (e.g. opinion) or objective (e.g. measurable by a test or diagnostic method).

In another embodiment, any of the above uses for treatment comprises the further step of co-administering to the subject in need thereof one or more second therapeutic agents. The choice of second therapeutic agent may be made from any second therapeutic agent known to be useful for co-administration with palbociclib. The choice of second therapeutic agent is also dependent upon the particular disease or condition to be treated. Examples of second therapeutic agents that may be employed in this invention are those set forth above for use in combination compositions comprising a compound of this invention and a second therapeutic agent.

In particular, the combination therapies of this invention include co-administering a compound of Formula I and a second therapeutic agent to a subject in need thereof for treatment of the following conditions (with the particular second therapeutic agent indicated in parentheses following the indication): colorectal cancer (5-FU and/or oxaliplatin); multiple myeloma (dexamethasone and/or bortezomib); breast cancer (anastrozole or letrozole or paclitaxel); mantle cell lymphoma (bortezomib).

The term "co-administered" as used herein means that the second therapeutic agent may be administered together with a compound of this invention as part of a single dosage form (such as a composition of this invention comprising a compound of the invention and an second therapeutic agent as described above) or as separate, multiple dosage forms. Alternatively, the additional agent may be administered prior to, consecutively with, or following the administration of a compound of this invention. In such combination therapy treatment, both the compounds of this invention and the second therapeutic agent(s) are administered by conventional methods. The administration of a composition of this invention, comprising both a compound of the invention and a second therapeutic agent, to a subject

does not preclude the separate administration of that same therapeutic agent, any other second therapeutic agent or any compound of this invention to said subject at another time during a course of treatment.

Effective amounts of these second therapeutic agents are well known to those skilled in the art and guidance for dosing may be found in patents and published patent applications referenced herein, as well as in Wells et al., eds., Pharmacotherapy Handbook, 2nd Edition, Appleton and Lange, Stamford, Conn. (2000); PDR Pharmacopoeia, Tarascon Pocket Pharmacopoeia 2000, Deluxe Edition, Tarascon Publishing, Loma Linda, Calif. (2000), and other medical texts. However, it is well within the skilled artisan's purview to determine the second therapeutic agent's optimal effective-amount range.

In one embodiment of the invention, where a second therapeutic agent is administered to a subject, the effective amount of the compound of this invention is less than its effective amount would be where the second therapeutic agent is not administered. In another embodiment, the effective amount of the second therapeutic agent is less than its effective amount would be where the compound of this invention is not administered. In this way, undesired side effects associated with high doses of either agent may be minimized. Other potential advantages (including without limitation improved dosing regimens and/or reduced drug cost) will be apparent to those of skill in the art.

In yet another aspect, the invention provides the use of a compound of Formula I alone or together with one or more of the above-described second therapeutic agents in the manufacture of a medicament, either as a single composition or as separate dosage forms, for treatment in a subject of a disease, disorder or symptom set forth above. Another aspect of the invention is a compound of Formula I for use in the treatment in a subject of a disease, disorder or symptom thereof delineated herein.

### Example 1. Evaluation of Metabolic Stability

***Microsomal Assay:*** Human liver microsomes (20 mg/mL) are obtained from Xenotech, LLC (Lenexa, KS). β-nicotinamide adenine dinucleotide phosphate, reduced form (NADPH), magnesium chloride ($MgCl_2$), and dimethyl sulfoxide (DMSO) are purchased from Sigma-Aldrich.

***Determination of Metabolic Stability:*** 7.5 mM stock solutions of test compounds are prepared in DMSO. The 7.5 mM stock solutions are diluted to 12.5-50 μM in acetonitrile (ACN). The 20 mg/mL human liver microsomes are diluted to 0.625 mg/mL in 0.1 M potassium phosphate buffer, pH 7.4, containing 3 mM $MgCl_2$. The diluted microsomes are added to wells of a 96-well deep-well polypropylene plate in triplicate. A 10 μL aliquot of the 12.5-50 μM test compound is added to the microsomes and the mixture is pre-warmed for 10 minutes. Reactions are initiated by addition of pre-warmed NADPH solution. The final reaction volume is 0.5 mL and contains 0.5 mg/mL human liver microsomes, 0.25-1.0 μM test compound, and 2 mM NADPH in 0.1 M potassium phosphate buffer, pH 7.4, and 3 mM $MgCl_2$. The reaction mixtures are incubated at 37 °C, and 50 μL aliquots are removed at 0,5, 10, 20, and 30 minutes and added to shallow-well 96-well plates which contain 50 μL of ice-cold ACN with internal standard to stop the reactions. The plates are stored at 4 °C for 20 minutes after which 100 μL of water is added to the wells of the plate before centrifugation to pellet precipitated proteins. Supernatants are transferred to another 96-well plate and analyzed for amounts of parent remaining by LC-MS/MS using an Applied Bio-systems API 4000 mass spectrometer. The same procedure is followed for the non-deuterated counterpart of the compound of Formula I and the positive control, 7-ethoxycoumarin (1 μM). Testing is done in triplicate.

***Data analysis:*** The *in vitro* $t_{1/2}$s for test compounds are calculated from the slopes of the linear regression of % parent remaining (ln) vs incubation time relationship.

$$\text{in vitro } t_{½} = 0.693/k$$

$$k = -[\text{slope of linear regression of \% parent remaining(ln) vs incubation time}]$$

Data analysis is performed using Microsoft Excel Software.

## Claims

**1.** A compound of Formula I:

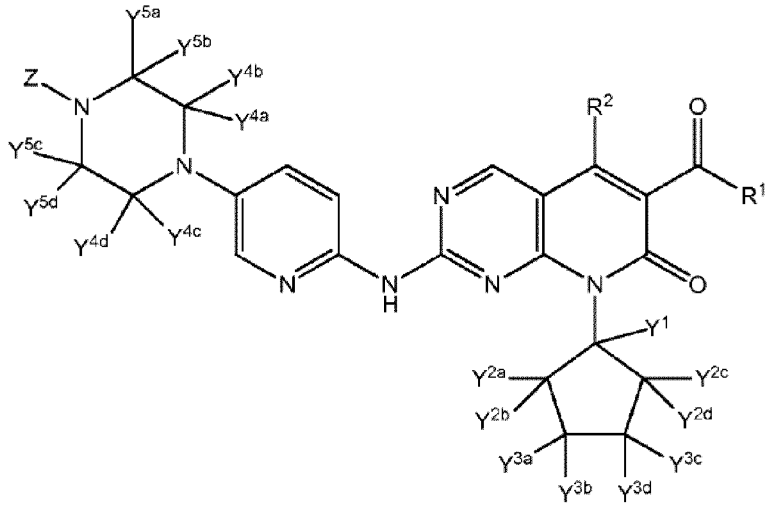

,

or a pharmaceutically acceptable salt thereof, wherein:

each of $Y^1$, $Y^{2a}$, $Y^{2b}$, $Y^{2c}$, $Y^{2d}$, $Y^{3a}$, $Y^{3b}$, $Y^{3c}$ and $Y^{3d}$ is independently hydrogen or deuterium;
each of $Y^{4a}$, $Y^{4b}$, $Y^{4c}$, $Y^{4d}$, $Y^{5a}$, $Y^{5b}$, $Y^{5c}$ and $Y^{5d}$ is deuterium;
each of $R^1$ and $R^2$ is independently selected from $CH_3$, $CH_2D$, $CHD_2$ and $CD_3$;
Z is selected from hydrogen, $-C(O)OCH_2OP(O)(OH)_2$,
and $-C(O)OCH_2OC(O)CH(R^3)NH_2$;
$R^3$ is selected from hydrogen, $-C_1$-$C_7$ alkyl, and $-(C_0$-$C_5$ alkylene)-$C_6$-$C_{10}$ aryl, wherein any alkyl, alkylene or aryl portion of $R^3$ is optionally substituted with -OH.

**2.** The compound of claim 1, wherein:

each of $Y^{2a}$ and $Y^{2b}$ is the same;
each of $Y^{2c}$ and $Y^{2d}$ is the same;
each of $Y^{3a}$ and $Y^{3b}$ is the same; and
each of $Y^{3c}$ and $Y^{3d}$ is the same.

**3.** The compound of claim 2, wherein:

each of $Y^{2a}$, $Y^{2b}$, $Y^{2c}$, and $Y^{2d}$ is the same; and
each of $Y^{3a}$, $Y^{3b}$, $Y^{3c}$, and $Y^{3d}$ is the same.

**4.** The compound of any one of claims 1-3, wherein each of $R^1$ and $R^2$ is independently selected from $CH_3$ and $CD_3$.

**5.** The compound of claim 4, wherein $R^1$ is $CH_3$ and $R^2$ is $CD_3$.

**6.** The compound of claim 4, wherein $R^1$ is $CD_3$ and $R^2$ is $CH_3$.

**7.** The compound of claim 4, wherein $R^1$ is $CD_3$ and $R^2$ is $CD_3$.

**8.** The compound of claim 4, wherein $R^1$ is $CH_3$ and $R^2$ is $CH_3$.

**9.** The compound of any one of claims 1-8, wherein Z is hydrogen.

**10.** The compound of claim 1, wherein Z is hydrogen; each of $Y^{2a}$, $Y^{2b}$, $Y^{2c}$, and $Y^{2d}$ is the same; each of $Y^{3a}$, $Y^{3b}$, $Y^{3c}$, and $Y^{3d}$ is the same; $R^1$ is $-CH_3$, and the compound is selected from any one of the compounds (Cmpd) set forth in the table (below):

| Cmpd # | $Y^1$ | $Y^{2a-d}$ | $Y^{3a-d}$ | $Y^{4a-d}$ | $Y^{5a-d}$ | $R^2$ |
|---|---|---|---|---|---|---|
| 115 | H | H | H | D | D | $CH_3$ |
| 121 | H | D | H | D | D | $CH_3$ |
| 122 | H | H | D | D | D | $CH_3$ |
| 123 | D | D | H | D | D | $CH_3$ |
| 124 | H | D | D | D | D | $CH_3$ |
| 125 | D | D | D | D | D | $CH_3$ |
| 139 | H | H | H | D | D | $CD_3$ |
| 145 | H | D | H | D | D | $CD_3$ |
| 146 | H | H | D | D | D | $CD_3$ |
| 147 | D | D | H | D | D | $CD_3$ |
| 148 | H | D | D | D | D | $CD_3$ |
| 149 | D | D | D | D | D | $CD_3$ |

wherein any atom not designated as deuterium is present at its natural isotopic abundance, or a pharmaceutically acceptable salt thereof.

**11.** The compound of claim 10, wherein the compound is selected from any one of the following compounds:

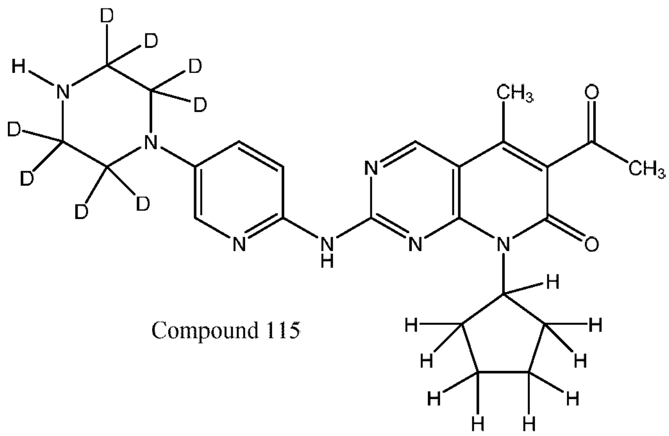

Compound 115

,

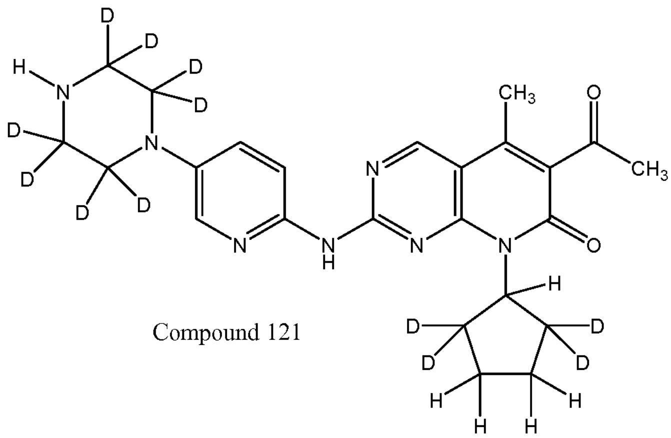

Compound 121

,

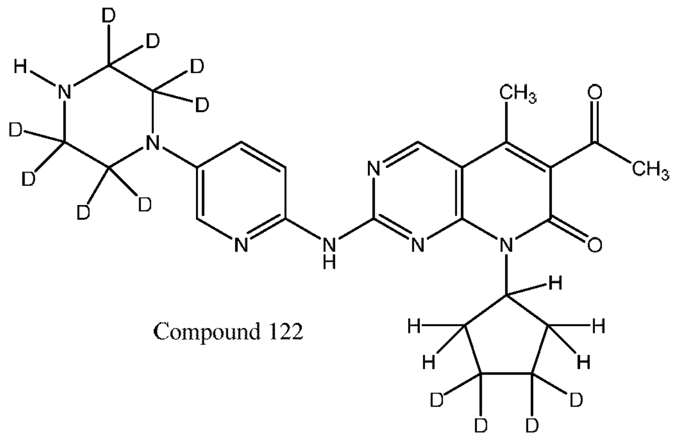

Compound 122

,

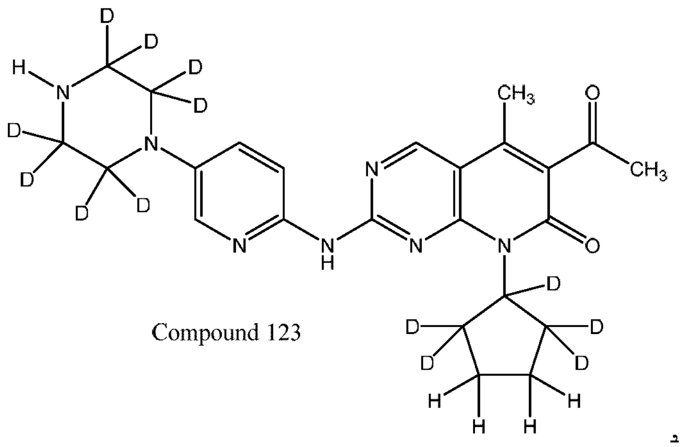

Compound 123

,

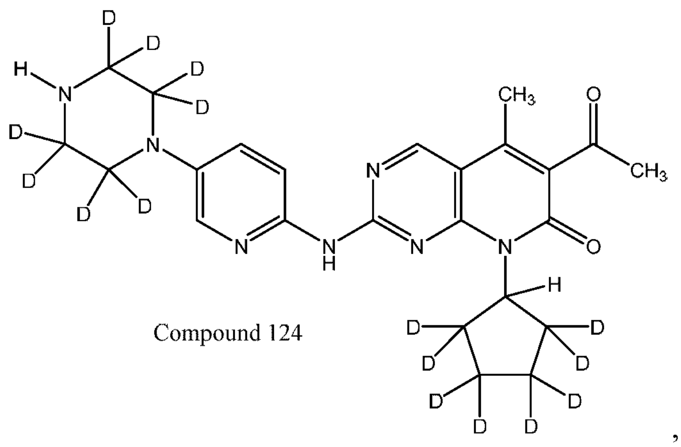

Compound 124

,

and

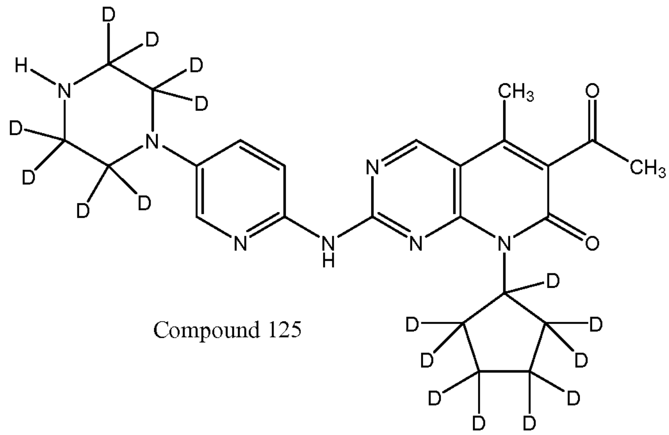

Compound 125

or a pharmaceutically acceptable salt thereof.

**12.** The compound of any one of claims 1-9, wherein any atom not designated as deuterium is present at its natural isotopic abundance.

**13.** The compound of any one of claims 1 to 12, wherein each position designated specifically as deuterium has at least 90% incorporation of deuterium at that position.

**14.** The compound of any one of claims 1 to 12, wherein each position designated specifically as deuterium has at least 95% incorporation of deuterium at that position.

**15.** A pharmaceutical composition comprising a compound of any one of claims 1-14; and a pharmaceutically acceptable carrier.

**16.** A composition of claim 15 for use in treating a disease or condition selected from cancer, autoimmune disease and allergy in a subject in need thereof.

**17.** The composition for use of claim 16, wherein the disease or condition is cancer and is selected from breast cancer, solid tumor, colorectal cancer, hepatocellular carcinoma, liposarcoma, ovarian cancer, multiple myeloma, acute leukemia, mantle cell lymphoma, myelodysplasia, glioblastoma, and non-small cell lung cancer.

**18.** The composition for use of claim 16 or 17, comprising the further step of co-administering to the subject in need thereof one or more second therapeutic agents, preferably wherein:

a. the disease is colorectal cancer and the second therapeutic agent is selected from one or more of 5-FU and oxaliplatin;
b. the disease is multiple myeloma and the second therapeutic agent is selected from one or more of dexamethasone and bortezomib;
c. the disease is breast cancer and the second therapeutic agent is selected from one or more of anastrozole, letrozole and paclitaxel; or
d. the disease is mantle cell lymphoma and the second therapeutic agent is bortezomib.

**Patentansprüche**

**1.** Verbindung nach Formel I:

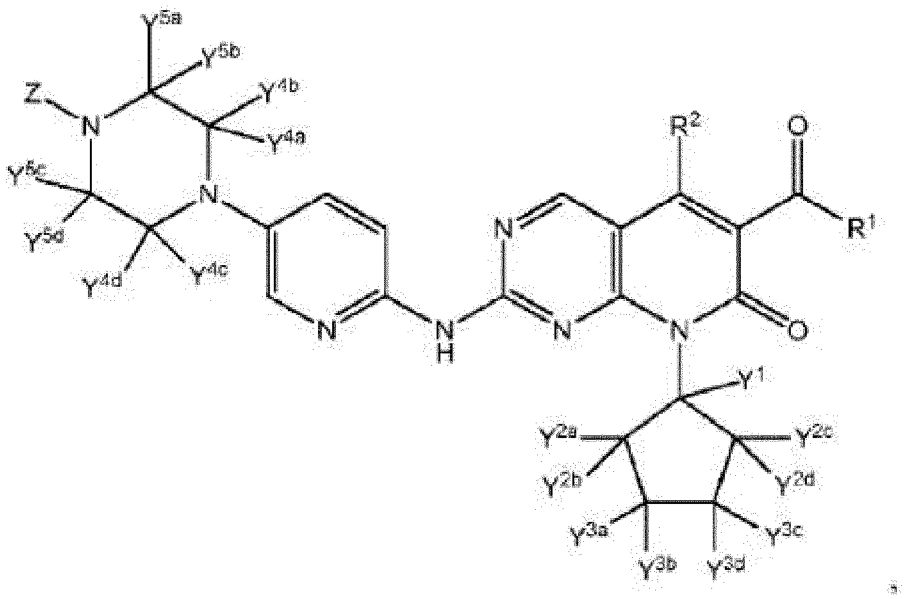

oder ein pharmazeutisch unbedenkliches Salz davon, wobei:

jedes aus $Y^1$, $Y^2$, $Y^2$, $Y^2$, $Y^2$, $Y^3$, $Y^3$, $Y^3$ und $Y^3$ unabhängig Wasserstoff oder Deuterium ist;
jedes aus $Y^4$, $Y^4$, $Y^4$, $Y^4$, $Y^5$, $Y^5$, $Y^5$ und $Y^5$ Deuterium ist;
jedes aus $R^1$ und $R^2$ unabhängig aus $CH_3$, $CH_2D$, $CHD_2$ und $CD_3$ ausgewählt ist;
Z aus Wasserstoff, $-O(O)OOH_2OP(O)(OH)_2$ und $-C(O)OCH_2OC(O)CH(R^3)NH_2$ ausgewählt ist;
$R^3$ aus Wasserstoff, $-C_1$-$C_7$-Alkyl und $-(C_0$-$C_5$-Alkylen)-$C_6$-$C_{10}$-Aryl ausgewählt ist, wobei jeder Alkyl-, Alkylen- oder Aryl-Abschnitt von $R^3$ optional durch -OH ersetzt ist.

**2.** Verbindung nach Anspruch 1, wobei:

jedes aus $Y^{2a}$ und $Y^{2b}$ gleich ist;
jedes aus $Y^{2c}$ und $Y^{2d}$ gleich ist;
jedes aus $Y^{3a}$ und $Y^{3b}$ gleich ist; und
jedes aus $Y^{3c}$ und $Y^{3d}$ gleich ist.

**3.** Verbindung nach Anspruch 2, wobei:

jedes aus $Y^{2a}$, $Y^{2b}$, $Y^{2c}$ und $Y^{2d}$ gleich ist; und
jedes aus $Y^{3a}$, $Y^{3b}$, $Y^{3c}$ und $Y^{3d}$ gleich ist.

**4.** Verbindung nach einem der Ansprüche 1-3, wobei jedes aus $R^1$ und $R^2$ unabhängig aus $CH_3$ und $CD_3$ ausgewählt ist.

**5.** Verbindung nach Anspruch 4, wobei $R^1$ $CH_3$ ist und $R^2$ $CD_3$ ist.

**6.** Verbindung nach Anspruch 4, wobei $R^1$ $CD_3$ ist und $R^2$ $CH_3$ ist.

**7.** Verbindung nach Anspruch 4, wobei $R^1$ $CD_3$ ist und $R^2$ $CD_3$ ist.

**8.** Verbindung nach Anspruch 4, wobei $R^1$ $CH_3$ ist und $R^2$ $CH_3$ ist.

**9.** Verbindung nach einem der Ansprüche 1-8, wobei Z Wasserstoff ist.

**10.** Verbindung nach Anspruch 1, wobei Z Wasserstoff ist; jedes aus $Y^{2a}$, $Y^{2b}$, $Y^{2c}$ und $Y^{2d}$ gleich ist; jedes aus $Y^{3a}$, $Y^{3b}$, $Y^{3c}$ and $Y^{3d}$ gleich ist; $R^1$ $-CH_3$ ist und die Verbindung aus einer der in der (nachfolgenden) Tabelle dargelegten Verbindungen (Vbdg) ausgewählt ist:

| Vbdg # | $Y^1$ | $Y^{2a-d}$ | $Y^{3a-d}$ | $Y^{4a-d}$ | $Y^{5a-d}$ | $R^2$ |
|---|---|---|---|---|---|---|
| 115 | H | H | H | D | D | $CH_3$ |
| 121 | H | D | H | D | D | $CH_3$ |
| 122 | H | H | D | D | D | $CH_3$ |
| 123 | D | D | H | D | D | $CH_3$ |
| 124 | H | D | D | D | D | $CH_3$ |
| 125 | D | D | D | D | D | $CH_3$ |
| 139 | H | H | H | D | D | $CD_3$ |
| 145 | H | D | H | D | D | $CD_3$ |
| 146 | H | H | D | D | D | $CD_3$ |
| 147 | D | D | H | D | D | $CD_3$ |
| 148 | H | D | D | D | D | $CD_3$ |
| 149 | D | D | D | D | D | $CD_3$ |

wobei jedes Atom, das nicht als Deuterium bezeichnet wird, mit seiner natürlichen Isotopenhäufigkeit vorliegt oder ein pharmazeutisch unbedenkliches Salz davon ist.

**11.** Verbindung nach Anspruch 10, wobei die Verbindung aus einer der folgenden Verbindungen ausgewählt ist:

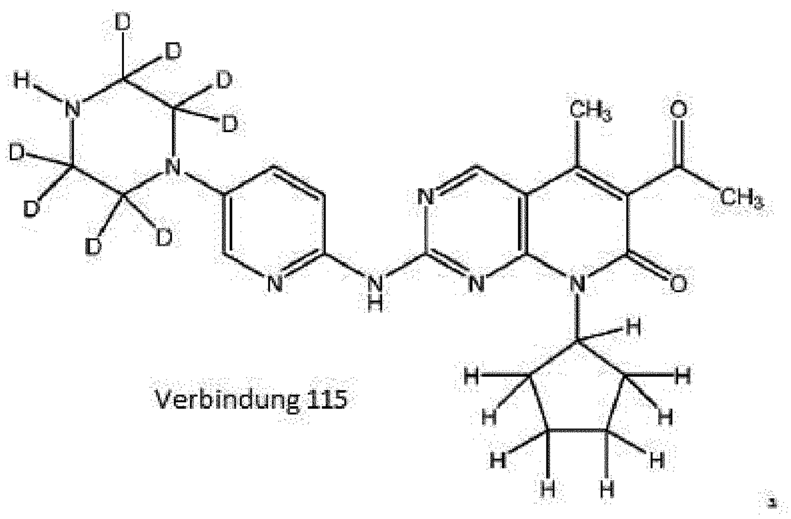

Verbindung 115

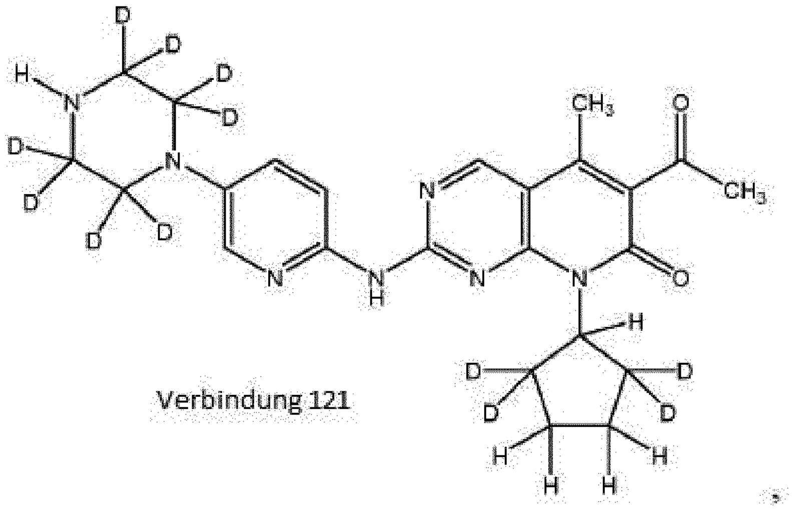

Verbindung 121

,

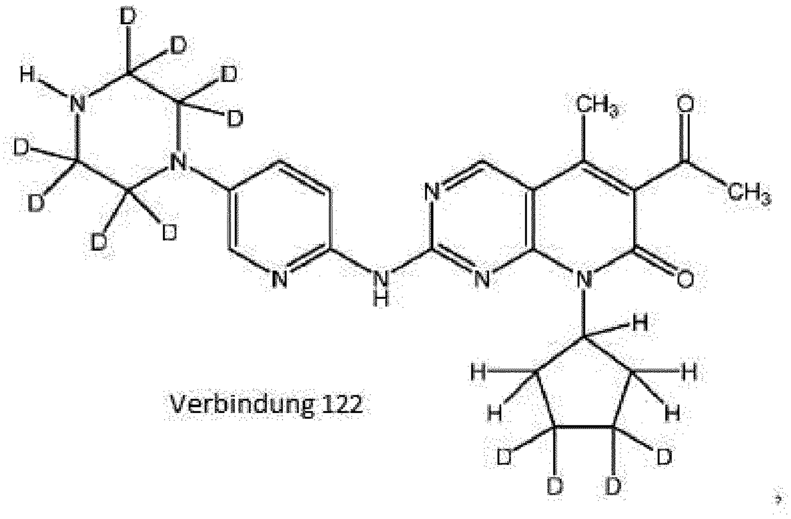

Verbindung 122

,

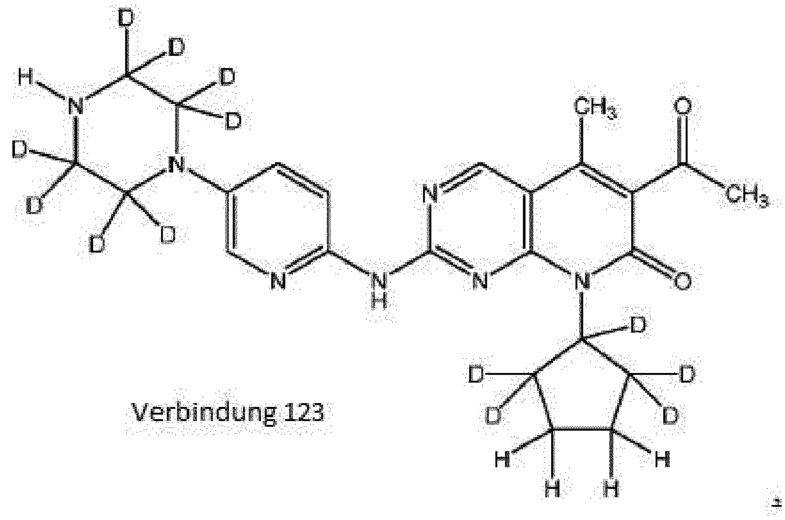

Verbindung 123

,

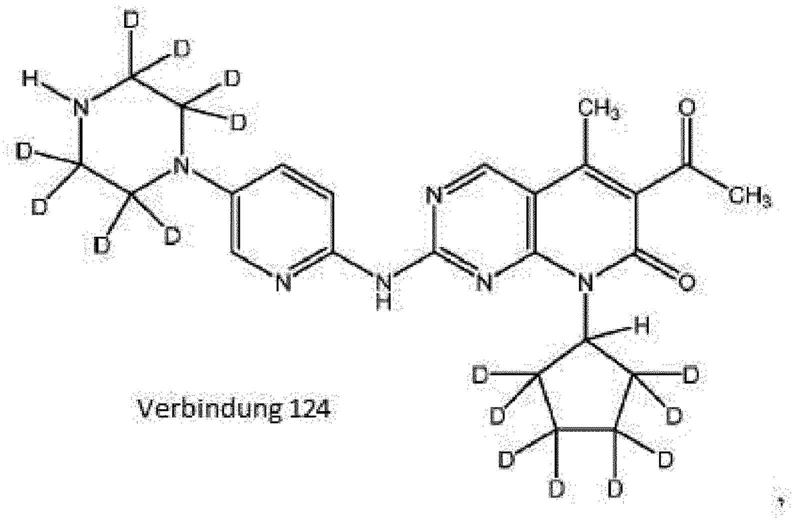

Verbindung 124

,

und

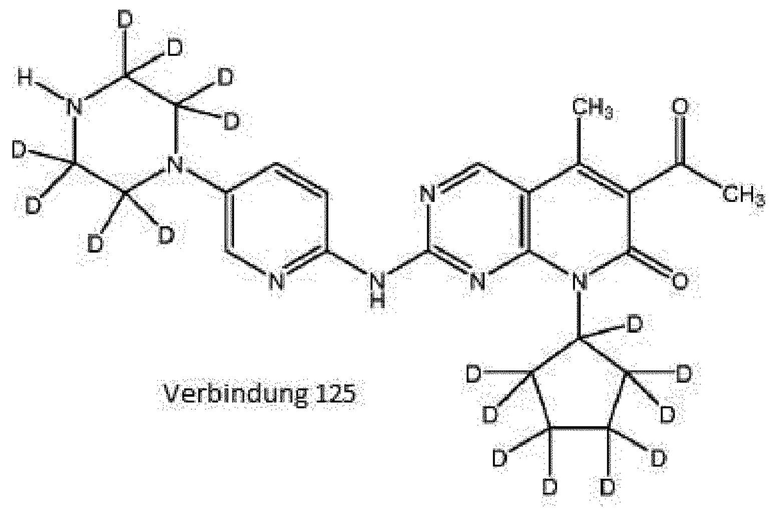

Verbindung 125

oder einem pharmazeutisch unbedenklichen Salz davon.

**12.** Verbindung nach einem der Ansprüche 1-9, wobei jedes Atom, das nicht als Deuterium bezeichnet wird, mit seiner natürlichen Isotopenhäufigkeit vorliegt.

**13.** Verbindung nach einem der Ansprüche 1 bis 12, wobei jede Position, die spezifisch als Deuterium bezeichnet wird, wenigstens 90 % Inkorporation von Deuterium an dieser Position besitzt.

**14.** Verbindung nach einem der Ansprüche 1 bis 12, wobei jede Position, die spezifisch als Deuterium bezeichnet wird, wenigstens 95 % Inkorporation von Deuterium an dieser Position besitzt.

**15.** Pharmazeutische Zusammensetzung umfassend eine Verbindung nach einem der Ansprüche 1-14; und einen pharmazeutisch unbedenklichen Träger.

**16.** Zusammensetzung nach Anspruch 15 zur Verwendung beim Behandeln einer Krankheit oder Erkrankung, ausgewählt aus Krebs, Autoimmunkrankheit und Allergie, bei einem behandlungsbedürftigen Subjekt.

**17.** Zusammensetzung zur Verwendung nach Anspruch 16, wobei die Krankheit oder Erkrankung Krebs ist und aus Brustkrebs, festem Tumor, kolorektalem Krebs, hepatozellulärem Karzinom, Liposarkom, Eierstockkrebs, multiplem Myelom, akuter Leukämie, Mantelzelllymphom, Myelodysplasie, Glioblastom und nichtkleinzelligem Lungenkrebs

ausgewählt ist.

**18.** Zusammensetzung zur Verwendung nach Anspruch 16 oder 17, umfassend den weiteren Schritt des zusätzlichen Verabreichens eines oder mehrerer zweiter therapeutischer Mittel dem behandlungsbedürftigen Subjekt, vorzugsweise wobei:

a. die Krankheit kolorektaler Krebs ist und das zweite therapeutische Mittel aus einem oder mehreren von 5-FU und Oxaliplatin ausgewählt ist;
b. die Krankheit ein multiples Myelom ist und das zweite therapeutische Mittel aus einem oder mehreren von Dexamethason und Bortezomib ausgewählt ist;
c. die Krankheit Brustkrebs ist und das zweite therapeutische Mittel aus einem oder mehreren von Anastrozol, Letrozol und Paclitaxel ausgewählt ist; oder
d. die Krankheit Mantelzelllymphom ist und das zweite therapeutische Mittel Bortezomib ist.

## Revendications

**1.** Composé de formule I :

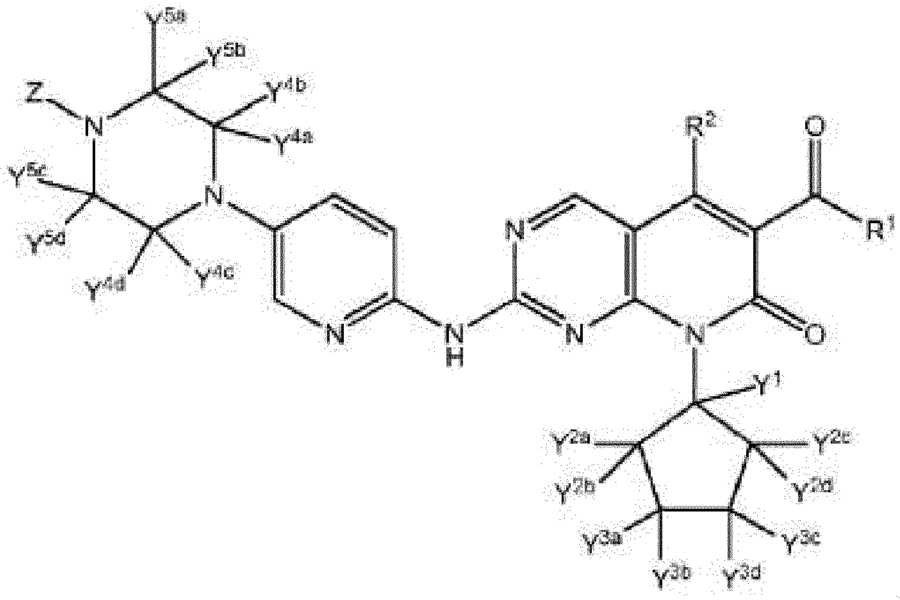

ou sel pharmaceutiquement acceptable de celui-ci, dans lequel :

chacun de $Y^1$, $Y^{2a}$, $Y^{2b}$, $Y^{2c}$, $Y^{2d}$, $Y^{3a}$, $Y^{3b}$, $Y^{3c}$ et $Y^{3d}$ est indépendamment de l'hydrogène ou du deutérium ;
chacun de $Y^{4a}$, $Y^{4b}$, $Y^{4c}$, $Y^{4d}$, $Y^{5a}$, $Y^{5b}$, $Y^{5c}$ et $Y^{5d}$ est du deutérium ;
chacun de $R^1$ et $R^2$ est indépendamment choisi parmi $CH_3$, $CH_2D$, $CHD_2$ et $CD_3$ ;
Z est choisi parmi l'hydrogène, $-C(O)OCH_2OP(O)(OH)_2$ et $-O(O)OOH_2OO(O)OH(R^3)NH_2$ ;
$R^3$ est choisi parmi l'hydrogène, l'alkyle en $C_1$-$C_7$ et l'(alkylène en $C_0$-$C_5$)-aryle en $C_6$-$C_{10}$, où une quelconque portion alkyle, alkylène ou aryle de $R^3$ est facultativement substituée par -OH.

**2.** Composé selon la revendication 1, dans lequel :

chacun de $Y^{2a}$ et $Y^{2b}$ est le même ;
chacun de $Y^{2c}$ et $Y^{2d}$ est le même ;
chacun de $Y^{3a}$ et $Y^{3b}$ est le même ; et
chacun de $Y^{3c}$ et $Y^{3d}$ est le même.

**3.** Composé selon la revendication 2, dans lequel :

chacun de $Y^{2a}$, $Y^{2b}$, $Y^{2c}$ et $Y^{2d}$ est le même ; et
chacun de $Y^{3a}$, $Y^{3b}$, $Y^{3c}$ et $Y^{3d}$ est le même.

**4.** Composé selon l'une quelconque des revendications 1 à 3, dans lequel chacun de $R^1$ et $R^2$ est indépendamment choisi parmi $CH_3$ et $CD_3$.

**5.** Composé selon la revendication 4, dans lequel $R^1$ est $CH_3$ et $R^2$ est $CD_3$.

**6.** Composé selon la revendication 4, dans lequel $R^1$ est $CD_3$ et $R^2$ est $CH_3$.

**7.** Composé selon la revendication 4, dans lequel $R^1$ est $CD_3$ et $R^2$ est $CD_3$.

**8.** Composé selon la revendication 4, dans lequel $R^1$ est $CH_3$ et $R^2$ est $CH_3$.

**9.** Composé selon l'une quelconque des revendications 1 à 8, dans lequel Z est de l'hydrogène.

**10.** Composé selon la revendication 1, dans lequel Z est de l'hydrogène ; chacun de $Y^{2a}$, $Y^{2b}$, $Y^{2c}$ et $Y^{2d}$ est le même ; chacun de $Y^{3a}$, $Y^{3b}$, $Y^{3c}$ et $Y^{3d}$ est le même ; $R^1$ est $-CH_3$, et le composé est choisi parmi l'un quelconque des composés (Cmpd) indiqués dans le tableau (ci-dessous) :

| Cmpd # | $Y^1$ | $Y^{2a-d}$ | $Y^{3a-d}$ | $Y^{4a-d}$ | $Y^{5a-d}$ | $R^2$ |
|---|---|---|---|---|---|---|
| 115 | H | H | H | D | D | $CH_3$ |
| 121 | H | D | H | D | D | $CH_3$ |
| 122 | H | H | D | D | D | $CH_3$ |
| 123 | D | D | H | D | D | $CH_3$ |
| 124 | H | D | D | D | D | $CH_3$ |
| 125 | D | D | D | D | D | $CH_3$ |
| 139 | H | H | H | D | D | $CD_3$ |
| 145 | H | D | H | D | D | $CD_3$ |
| 146 | H | H | D | D | D | $CD_3$ |
| 147 | D | D | H | D | D | $CD_3$ |
| 148 | H | D | D | D | D | $CD_3$ |
| 149 | D | D | D | D | D | $CD_3$ |

dans lequel un quelconque atome non désigné comme étant du deutérium est présent à son abondance isotopique naturelle, ou sel pharmaceutiquement acceptable de celui-ci.

**11.** Composé selon la revendication 10, dans lequel le composé est choisi parmi l'un quelconque des composés suivants :

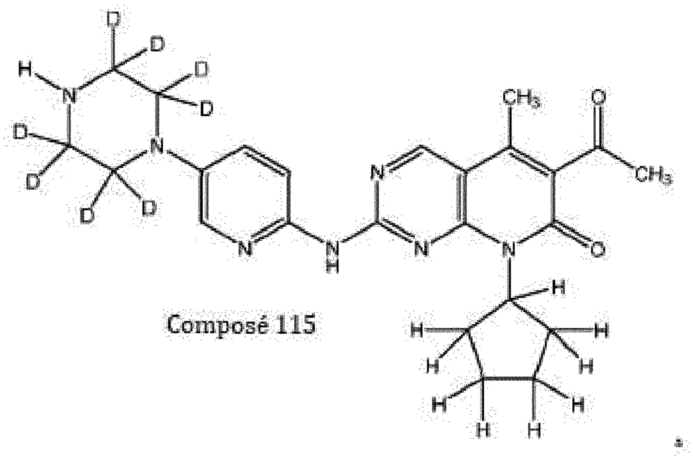

Composé 115

,

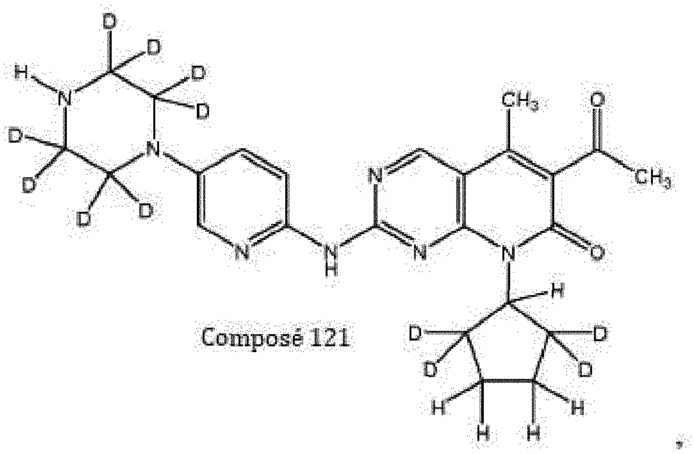

Composé 121

,

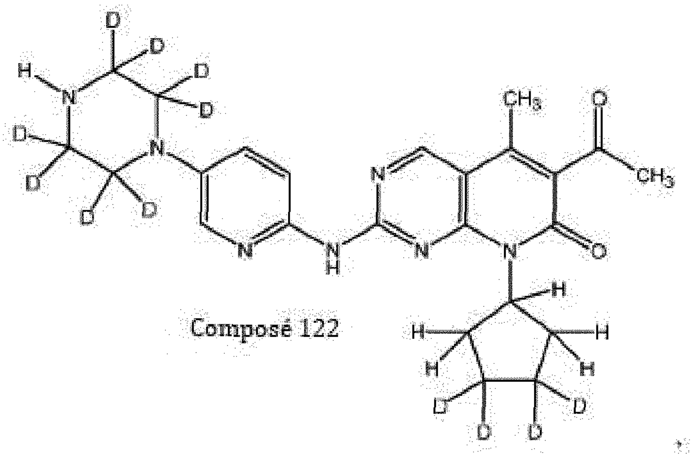

Composé 122

,

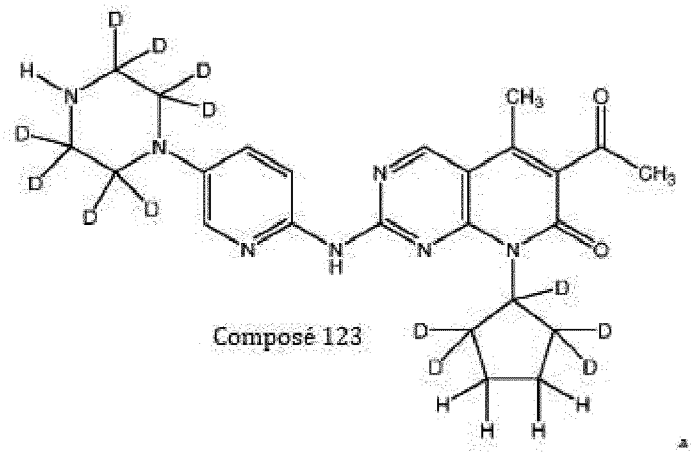

Composé 123,

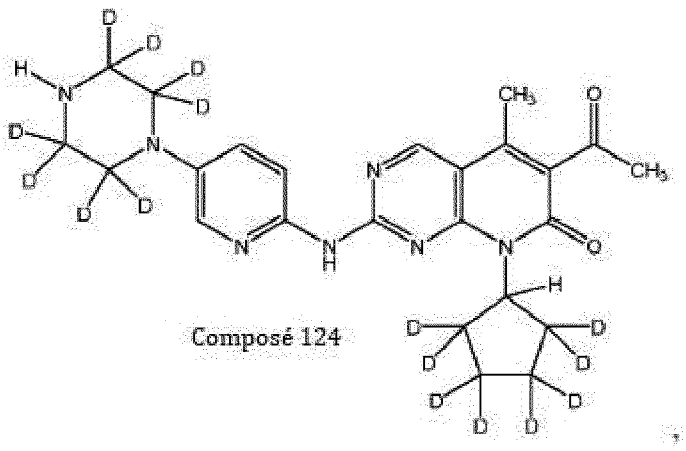

Composé 124,

et

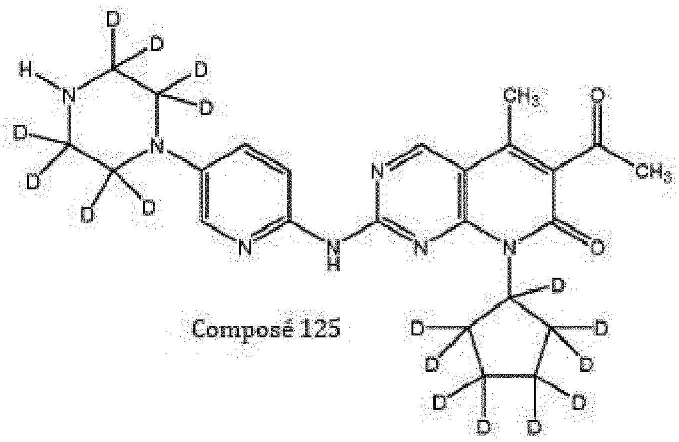

Composé 125

ou sel pharmaceutiquement acceptable de celui-ci.

**12.** Composé selon l'une quelconque des revendications 1 à 9, dans lequel un quelconque atome non désigné comme étant du deutérium est présent à son abondance isotopique naturelle.

**13.** Composé selon l'une quelconque des revendications 1 à 12, dans lequel chaque position spécifiquement désignée comme étant du deutérium a au moins 90 % d'incorporation de deutérium au niveau de cette position.

**14.** Composé selon l'une quelconque des revendications 1 à 12, dans lequel chaque position spécifiquement désignée

comme étant du deutérium a au moins 95 % d'incorporation de deutérium au niveau de cette position.

**15.** Composition pharmaceutique comprenant un composé selon l'une quelconque des revendications 1 à 14 ; et un support pharmaceutiquement acceptable.

**16.** Composition selon la revendication 15 pour une utilisation dans le traitement d'une maladie ou d'une condition choisie parmi le cancer, la maladie auto-immune et l'allergie chez un patient en ayant besoin.

**17.** Composition pour une utilisation selon la revendication 16, dans laquelle la maladie ou la condition est le cancer et est choisie parmi le cancer du sein, la tumeur solide, le cancer colorectal, le carcinome hépatocellulaire, le liposarcome, le cancer de l'ovaire, le myélome multiple, la leucémie aiguë, le lymphome à cellules du manteau, la myélodysplasie, le glioblastome et le cancer du poumon non à petites cellules.

**18.** Composition pour une utilisation selon la revendication 16 ou 17, comprenant l'étape supplémentaire de co-administration au patient en ayant besoin d'un ou de plusieurs agents thérapeutiques, de préférence dans laquelle :

a. la maladie est le cancer colorectal et le deuxième agent thérapeutique est choisi parmi un ou plusieurs du 5-FU et de l'oxaliplatine ;
b. la maladie est le myélome multiple et le deuxième agent thérapeutique est choisi parmi un ou plusieurs de la dexaméthasone et du bortézomib ;
c. la maladie est le cancer du sein et le deuxième agent thérapeutique est choisi parmi un ou plusieurs de l'anastrazole, du létrozole et du paclitaxel ; ou
d. la maladie est le lymphome à cellules du manteau et le deuxième agent thérapeutique est le bortézomib.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61787540 A
- WO 2011101417 A
- WO 2010039997 A
- WO 2010039997 A2
- WO 2007050732 A1
- EP 747385 A
- US 7014866 B
- US 20060094744 A
- US 20060079502 A
- US 6803031 B, Rabinowitz JD and Zaffaroni AC
- US 6099562 A
- US 5886026 A
- US 5304121 A
- WO 2003062236 A
- WO 2008076946 A
- WO 2009014642 A
- WO 2009061345 A
- WO 2010051127 A
- WO 2010132725 A
- WO 2011130232 A
- WO 2012068381 A
- WO 03062236 A


### Non-patent literature cited in the description


- **KEMPF, D.J. et al.** *Antimicrobial agents and chemotherapy,* 1997, vol. 41 (3), 654-60
- **WANG, L et al.** *Clinical Pharmacology and Therapeutics,* 1994, vol. 56 (6), 659-67
- **BLAKE, MI et al.** *J Pharm Sci,* 1975, vol. 64, 367-91
- **FOSTER, AB.** *Adv Drug Res,* 1985, vol. 14, 1-40
- **KUSHNER, DJ et al.** *Can J Physiol Pharmacol,* 1999, 79-88
- **FISHER, MB et al.** *Curr Opin Drug Discov Devel,* 2006, vol. 9, 101-09
- **FUKUTO et al.** *J. Med. Chem.,* 1991, vol. 34, 2871-76
- **FRY et al.** *Mol Cancer Ther,* 2004, vol. 3 (11
- **FINN et al.** *Breast Cancer Research,* vol. 11 (5
- **WADA, E et al.** *Seikagaku,* 1994, vol. 66, 15
- **GANNES, LZ et al.** *Comp Biochem Physiol Mol Integr Physiol,* 1998, vol. 119, 725
- **MIRIYALA, B. et al.** *Tetrahedron,* 2004, vol. 60, 1463-1471
- **DISCHINO, D. D. et al.** *Journal of Labelled Compounds and Radiopharmaceuticals,* 1988, vol. 25, 359-367
- **LAROCK R.** Comprehensive Organic Transformations. VCH Publishers, 1989
- **GREENE, TW et al.** Protective Groups in Organic Synthesis. John Wiley and Sons, 1999
- **FIESER, L et al.** Fieser and Fieser's Reagents for Organic Synthesis. John Wiley and Sons, 1994
- Encyclopedia of Reagents for Organic Synthesis. John Wiley and Sons, 1995
- Oral Lipid-Based Formulations: Enhancing the Bioavailability of Poorly Water-Soluble Drugs. Drugs and the Pharmaceutical Sciences. Informa Healthcare, 2007
- Role of Lipid Excipients in Modifying Oral and Parenteral Drug Delivery. Basic Principles and Biological Examples. Wiley-Interscience, 2006
- Remington: The Science and Practice of Pharmacy. Lippincott Williams & Wilkins, 2000
- **FREIREICH et al.** *Cancer Chemother. Rep,* 1966, vol. 50, 219
- *Scientific Tables, Geigy Pharmaceuticals,* 1970, 537
- Pharmacotherapy Handbook. Appleton and Lange, 2000
- PDR Pharmacopoeia, Tarascon Pocket Pharmacopoeia 2000. Tarascon Publishing, 2000